(19) Europäisches Patentamt
European Patent Office
Office européen des brevets

(11) **EP 4 781 919 A1**

(12) **EUROPEAN PATENT APPLICATION**
published in accordance with Art. 153(4) EPC

(43) Date of publication:
**29.07.2026  Bulletin 2026/31**

(21) Application number: 23958299.2

(22) Date of filing: **09.11.2023**

(51) International Patent Classification (IPC):
**A61B 10/00** *(2006.01)*     **A61B 5/11** *(2006.01)*
**F24F 11/63** *(2018.01)*     **F24F 120/14** *(2018.01)*

(52) Cooperative Patent Classification (CPC):
**A61B 5/11; A61B 10/00; F24F 11/63;** F24F 2120/14

(86) International application number:
**PCT/JP2023/040365**

(87) International publication number:
**WO 2025/099898 (15.05.2025 Gazette 2025/20)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Mitsubishi Electric Corporation**
**Tokyo 100-8310 (JP)**

(72) Inventors:
• **WATANABE, Futa**
  **Tokyo 100-8310 (JP)**
• **OSAWA, Risa**
  **Tokyo 100-8310 (JP)**

(74) Representative: **Pfenning, Meinig & Partner mbB**
**Patent- und Rechtsanwälte**
**Theresienhöhe 11a**
**80339 München (DE)**

(54) **THERMAL SENSATION ESTIMATION APPARATUS, THERMAL SENSATION ESTIMATION METHOD, AND THERMAL SENSATION ESTIMATION PROGRAM**

(57)     A walking speed scale reception unit (111) receives a walking speed scale inputted as a scale that represents a walking speed of a user. A walking time acquisition unit (120) acquires walking time information representing, as walking time, a duration of time for which the user was walking. A hot/cold sensation estimation unit (130) estimates, using the received walking speed scale and the acquired walking time information as usage information, hot/cold sensation felt by the user after walking based on the usage information.

Fig.1

## Description

## Technical Field

The present disclosure relates to a technology for estimating hot/cold sensation after walking of a user.

## Background Art

Patent Literature 1 discloses a technology for controlling an air conditioner.

In this technology, when a user (subject) returns indoors after being active outdoors, outdoor travel data (traveling amount, traveling speed) is collected. Next, a metabolic rate of the user is calculated based on the collected data. Then, an air conditioner is controlled such that a PMV (comfort evaluation value) based on the metabolic rate of the user, an air flow temperature of the air conditioner, an air flow humidity of the air conditioner, and so on falls within a comfortable range.

The outdoor travel data is collected using location information of a smartphone.

The location information of the smartphone is obtained through a satellite positioning system (such as GPS and GNSS ), but includes an error ranging from 1 meter to about 100 meters.

Therefore, an accuracy of the travel data obtained using the location information of the smartphone is low.

Note that GPS is short for Global Positioning System.

Note that GNSS is short for Global Navigation Satellite System.

## Citation List

## Patent Literature

Patent Literature 1: WO 2008/087959

## Summary of Invention

## Technical Problem

In the technology of Patent Literature 1, because of a low accuracy of travel data used, it is not possible to accurately calculate a metabolic rate of a user. Therefore, it is not possible to optimally control an air conditioner for the benefit of the user.

An objective of the present disclosure is to enable more accurate estimation of hot/cold sensation felt by a user after walking.

## Solution to Problem

A hot/cold sensation estimation device of the present disclosure includes:

a walking speed scale reception unit to receive a walking speed scale inputted as a scale that represents a walking speed of a user;
a walking time acquisition unit to acquire walking time information representing, as walking time, a duration of time for which the user was walking; and
a hot/cold sensation estimation unit to estimate, using the received walking speed scale and the acquired walking time information as usage information, hot/cold sensation felt by the user after walking based on the usage information. **Advantageous Effects of Invention**

According to the present disclosure, it is possible to more accurately estimate hot/cold sensation felt by a user after walking.

## Brief Description of Drawings

Fig. 1 is a configuration diagram of a hot/cold sensation estimation device 100 in Embodiment 1.
Fig. 2 is a flowchart of a hot/cold sensation estimation method in Embodiment 1.
Fig. 3 is a diagram to describe a walking speed based on location information.
Fig. 4 is a configuration diagram of a hot/cold sensation estimation device 100 in Embodiment 2.
Fig. 5 is a flowchart of a hot/cold sensation estimation method in Embodiment 2.
Fig. 6 is a configuration diagram of a hot/cold sensation estimation device 100 in Embodiment 3.
Fig. 7 is a flowchart of a hot/cold sensation estimation method in Embodiment 3.
Fig. 8 is a configuration diagram of a hot/cold sensation estimation device 100 in an example of Embodiment 3.
Fig. 9 is a flowchart of a hot/cold sensation estimation method in the example of Embodiment 3.
Fig. 10 is a configuration diagram of a hot/cold sensation estimation device 100 in Embodiment 4.
Fig. 11 is a flowchart of a hot/cold sensation estimation method in Embodiment 4.
Fig. 12 is a configuration diagram of a hot/cold sensation estimation device 100 in an example of Embodiment 4.
Fig. 13 is a flowchart of a hot/cold sensation estimation method in the example of Embodiment 4.
Fig. 14 is a configuration diagram of a hot/cold sensation estimation device 100 in Embodiment 5.
Fig. 15 is a flowchart of a hot/cold sensation estimation method in Embodiment 5.
Fig. 16 is a configuration diagram of a hot/cold sensation estimation device 100 in Embodiment 6.
Fig. 17 is a flowchart of a hot/cold sensation estimation method in Embodiment 6.
Fig. 18 is a configuration diagram of a hot/cold sensation estimation device 100 in an example of Embodiment 6.

Fig. 19 is a flowchart of a hot/cold sensation estimation method in the example of Embodiment 6.

Fig. 20 is a configuration diagram of a hot/cold sensation estimation device 100 in Embodiment 7.

Fig. 21 is a flowchart of a hot/cold sensation estimation method in Embodiment 7.

Fig. 22 is a diagram illustrating an instance of a walking zone in Embodiment 7.

Fig. 23 is a diagram illustrating an instance of a walking zone in Embodiment 7.

Fig. 24 is a configuration diagram of a hot/cold sensation estimation device 100 in an example of Embodiment 7.

Fig. 25 is a flowchart of a hot/cold sensation estimation method in the example of Embodiment 7.

Fig. 26 is a configuration diagram of a hot/cold sensation estimation device 100 in Embodiment 8.

Fig. 27 is a flowchart of a hot/cold sensation estimation method in Embodiment 8.

Fig. 28 is a diagram illustrating an instance of a walking start zone in Embodiment 8.

Fig. 29 is a configuration diagram of a hot/cold sensation estimation device 100 in an example of Embodiment 8.

Fig. 30 is a flowchart of a hot/cold sensation estimation method in the example of Embodiment 8.

Fig. 31 is a configuration diagram of a hot/cold sensation estimation device 100 in Embodiment 9.

Fig. 32 is a flowchart of positioning function control (A) in Embodiment 9.

Fig. 33 is a flowchart of positioning function control (B) in Embodiment 9.

Fig. 34 is a configuration diagram of a hot/cold sensation estimation device 100 in an example of Embodiment 9.

Fig. 35 is a configuration diagram of a hot/cold sensation estimation device 100 in Embodiment 10.

Fig. 36 is a flowchart of a hot/cold sensation estimation method in Embodiment 10.

Fig. 37 is a configuration diagram of a hot/cold sensation estimation device 100 in an example of Embodiment 10.

Fig. 38 is a flowchart of a hot/cold sensation estimation method in the example of Embodiment 10.

Fig. 39 is a configuration diagram of a hot/cold sensation estimation device 100 in Embodiment 11.

Fig. 40 is a flowchart of a hot/cold sensation estimation method in Embodiment 11.

Fig. 41 is a configuration diagram of a hot/cold sensation estimation device 100 in an example of Embodiment 11.

Fig. 42 is a flowchart of a hot/cold sensation estimation method in the example of Embodiment 11.

Fig. 43 is a configuration diagram of a hot/cold sensation estimation device 100 in Embodiment 12.

Fig. 44 is a flowchart of a hot/cold sensation estimation method in Embodiment 12.

Fig. 45 is a configuration diagram of a hot/cold sensation estimation device 100 in an example of Embodiment 12.

Fig. 46 is a flowchart of a hot/cold sensation estimation method in the example of Embodiment 12.

Fig. 47 is a hardware configuration diagram of the hot/cold sensation estimation device 100 in the Embodiments.

## Description of Embodiments

In the Embodiments and drawings, the same elements or equivalent elements are denoted by the same reference sign. Description of an element denoted by the same reference sign as that of an element that has been described will be suitably omitted or simplified. Arrows in diagrams mainly indicate flows of data or flows of processing.

Embodiment 1.

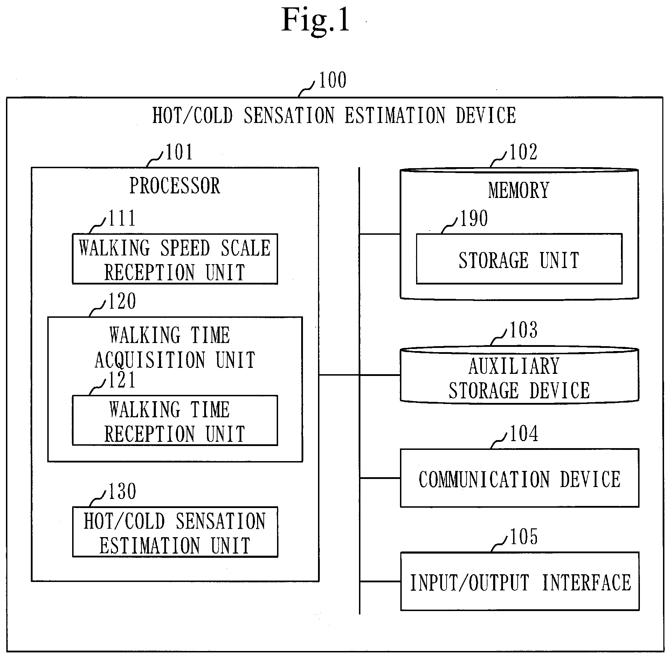

An embodiment for estimating hot/cold sensation felt by a user after walking will be described with referring to Fig. 1 through Fig. 3.

*** Description of Configuration ***

With referring to Fig. 1, a configuration of a hot/cold sensation estimation device 100 will be described.

The hot/cold sensation estimation device 100 is realized by a user terminal possessed by the user while user walks. Instances of the user terminal include a smartphone and a wearable device. An instance of the wearable device is a smart watch.

The hot/cold sensation estimation device 100 is a computer provided with hardware components such as a processor 101, a memory 102, an auxiliary storage device 103, a communication device 104, and an input/output interface 105. These hardware components are connected to each other through a signal line.

The processor 101 is an IC to perform computation processing, and controls other hardware components. The processor 101 is, for example, a CPU.

Note that IC is short for Integrated Circuit.

Note that CPU is short for Central Processing Unit.

The memory 102 is a volatile or non-volatile storage device. The memory 102 is also referred to as the main storage device or main memory. For example, the memory 102 is a RAM. Data stored in the memory 102 is saved in the auxiliary storage device 103 as needed.

Note that RAM is short for Random Access Memory.

The auxiliary storage device 103 is a non-volatile storage device. For example, the auxiliary storage device 103 can be a ROM, an HDD, a flash memory, or a combination of these. Data stored in the auxiliary storage device 103 is loaded into the memory 102 as needed.

Note that ROM is short for Read Only Memory.

Note that HDD is short for Hard Disk Drive.

The communication device 104 consists of a receiver and a transmitter. For example, the communication device 104 is a communication chip or an NIC. The hot/cold sensation estimation device 100 performs communication using the communication device 104.

Note that NIC is short for Network Interface Card.

The input/output interface 105 consists of an input device, an output device, and an input/output port. For example, the input device consists of a touch panel and a microphone, and the output device consists of a display and a speaker. Input to and output from the hot/cold sensation estimation device 100 are performed using the input/output interface 105.

The hot/cold sensation estimation device 100 includes elements such as a walking speed scale reception unit 111, a walking time acquisition unit 120, and a hot/cold sensation estimation unit 130. The walking time acquisition unit 120 is provided with a walking time reception unit 121. These elements are implemented by software.

The auxiliary storage device 103 stores a hot/-cold sensation estimation program designed that causes the computer to function as the walking speed scale reception unit 111, the walking time acquisition unit 120, and the hot/cold sensation estimation unit 130. The hot/cold sensation estimation program is loaded into the memory 102 and executed by the processor 101.

The auxiliary storage device 103 additionally stores an OS. At least part of the OS is loaded into the memory 102 and executed by the processor 101.

While executing the OS, the processor 101 also executes the hot/cold sensation estimation program.

Note that OS is short for Operating System.

Input/output data of the hot/cold sensation estimation program is stored in a storage unit 190.

The memory 102 functions as the storage unit 190. However, a storage device such as the auxiliary storage device 103, a register in the processor 101, and a cache memory in the processor 101 may function as the storage unit 190, either in place of, or together with, the memory 102.

The hot/cold sensation estimation program can be computer-readably recorded (stored) on a non-volatile storage medium such as an optical disc and a flash memory.

*** Description of Operation ***

An operation procedure of the hot/cold sensation estimation device 100 corresponds to a hot/cold sensation estimation method. The operation procedure of the hot/cold sensation estimation device 100 also corresponds a procedure for processing by the hot/cold sensation estimation program.

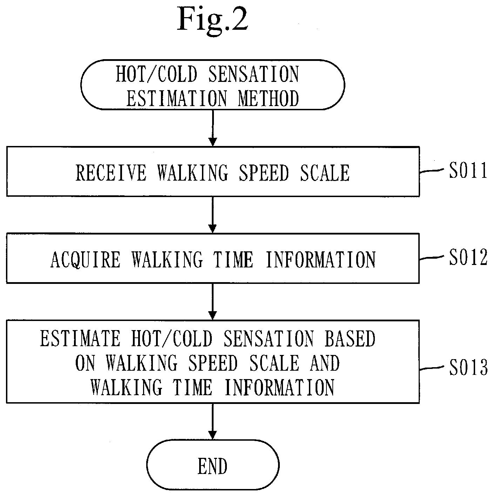

With referring to Fig. 2, the hot/cold sensation estimation method will be described.

In step S011, the walking speed scale reception unit 111 receives a walking speed scale inputted to the hot/cold sensation estimation device 100.

The walking speed scale is a scale that represents a walking speed of the user. The walking speed is a speed of the user in walking.

For example, the walking speed scale is expressed by an ordinal scale with three levels: "slow", "normal", and "fast". However, the walking speed scale could also be expressed by an ordinal scale with two levels or an ordinal scale with four or more levels.

The walking speed scale is inputted using a user interface. That is, the walking speed scale is a value declared by the user (declaration value).

For example, the walking speed scale is received as follows.

First, the user operates the user terminal after walking to activate the hot/cold sensation estimation device 100.

Next, the walking speed scale reception unit 111 displays a scale input window on the display. The scale input window has a graphical user interface (GUI) for inputting the walking speed scale.

Subsequently, the user operates the user terminal to input the walking speed scale to the scale input window.

Then, the walking speed scale reception unit 111 receives the inputted walking speed scale.

In step S012, the walking time acquisition unit 120 acquires walking time information.

The walking time information indicates walking time.

The walking time represents a duration of time for which the user was walking. Specifically, the walking time is a total amount of time for which the user was walking.

Specifically, the walking time reception unit 121 receives the walking time information inputted to the hot/cold sensation estimation device 100.

The walking time information is inputted using the user interface. In other words, the walking time is a value declared by the user (declaration value).

For example, the walking time information is received as follows.

First, the user operates the user terminal after walking to activate the hot/cold sensation estimation device 100.

Next, the walking time reception unit 121 displays a time input window on the display. The time input window has a graphical user interface (GUI) for inputting the walking time.

Next, the user operates the user terminal to input the walking time information to the time input window.

Then, the walking time reception unit 121 receives the inputted walking time information.

Step S011 and step S012 can be executed in a reverse order.

After step S011 and step S012, the processing proceeds to step S013.

In step S013, the hot/cold sensation estimation unit 130, using the received walking speed scale and the acquired walking time information as usage information, estimates the hot/cold sensation felt by the user after walking based on the usage information.

The hot/cold sensation is estimated by taking the usage information as input and using an estimation model.

A multiple regression model, a machine learning model, or the like is utilized as the estimation model.

When a machine learning model is utilized, a support vector machine, a random forest, an XGBoost, or the like is employed.

The hot/cold sensation may be expressed in real numbers or by an ordinal scale.

For example, the hot/cold sensation is expressed by a numerical value from 1 to N.

For example, the hot/cold sensation is expressed by an ordinal scale of N levels including "cold", "normal", and "hot".

After step S013, the processing of the hot/cold sensation estimation method ends.

*** Effects of Embodiment 1 ***

Embodiment 1 uses the walking speed scale (a value declared by the user) instead of walking speed based on location information, so that more likely hot/cold sensation felt by the user after walking can be estimated.

The estimated hot/cold sensation can be used, for example, to optimally control an indoor unit of a packaged air conditioner (PAC).

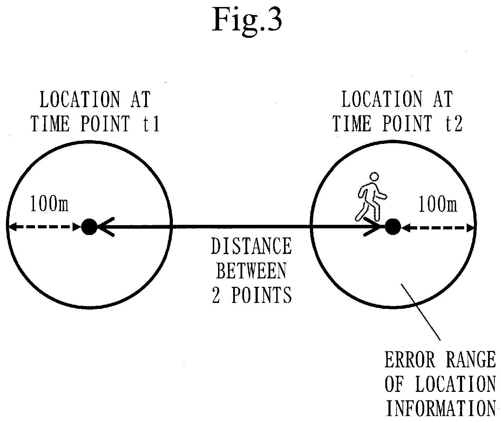

With referring to Fig. 3, the walking speed based on the location information will be described.

The walking speed is calculated as follows using the location information. The location information is obtained by GPS, GNSS, or the like.

　　(1) A distance between two points is calculated based on location information of the two points, and a walking speed between the two points is calculated based on the distance between the two points and the walking time between the two points. Then, walking speeds from a walking start point to a walking end point are averaged.
　　(2) The distance between the two points is calculated based on the location information of the two points, and a total walking distance is calculated by accumulating the distances between the two points. Then, the total walking distance is divided by the total walking time to calculate the walking speed.

The accuracy of the location information ranges from 1 meter to about 100 meters. Therefore, there may be an error of 200 meters at maximum in the distance between two points. In other words, in (1), there is a possibility that the accuracy of the walking speed between the two points is low. If location information is obtained once every 10 seconds, there may be an error of 1200 meters at maximum per minute in the total walking distance. In other words, in (2), there is a possibility that the accuracy of the walking speed is low.

The user is not always walking, and may stop to wait for a traffic signal and so on. Therefore, to determine the walking time or total walking time, whether the user is walking or is stopped is decided using the location information. However, as mentioned above, the accuracy of the walking speed may be low, so it is considered difficult to decide whether the user is walking or is stopped based on threshold judgment or the like. Also, whether the user is walking or is stopped may be decided using an acceleration sensor in the terminal. However, a measurement value of the acceleration sensor depends on the model or individual acceleration sensor, and use of the acceleration sensor will increase a power consumption of the terminal. Therefore, the method that uses the acceleration sensor is considered unrealistic.

As such, it is considered inappropriate to use walking speed based on location information.

In view of this, Embodiment 1 estimates hot/cold sensation felt after walking based on the walking speed scale (ordinal scale such as slow, normal, fast, and so on) of the user and the walking time of the user.

While the walking speed scale depends on the subjectivity of the user as it is a declaration value, it is possible to obtain a unique walking speed scale for every user. Therefore, it is possible to estimate more likely hot/cold sensation felt after walking. Moreover, regardless of the terminal used and no matter who uses which smartphone, it is possible to estimate the hot/cold sensation felt after walking. Furthermore, it is possible to estimate the hot/cold sensation felt after walking while suppressing power consumption of the terminal.

Embodiment 2.

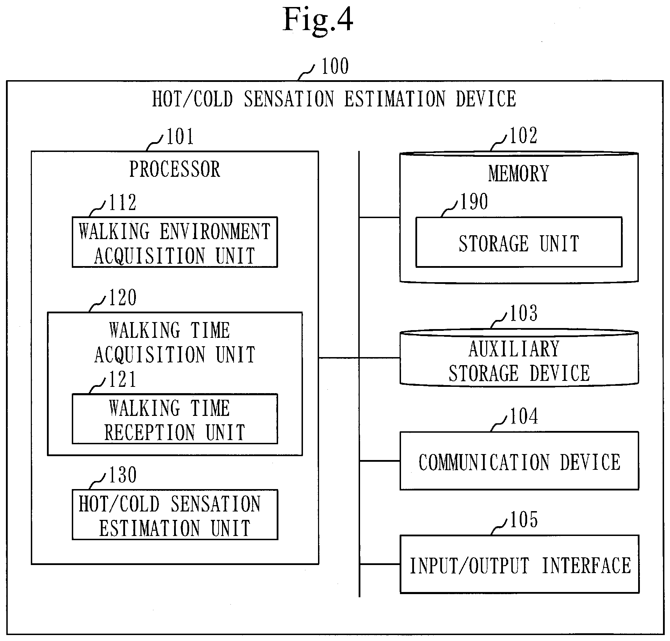

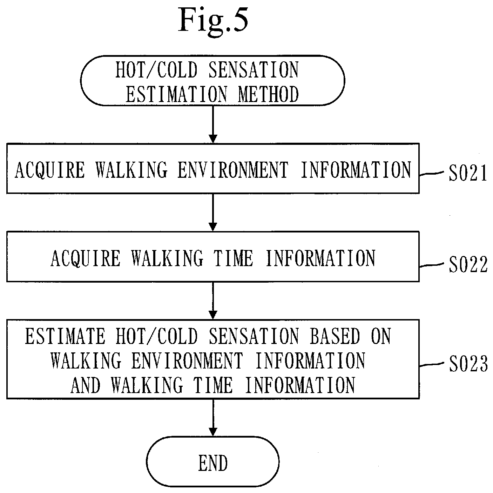

An embodiment for estimating hot/cold sensation felt after walking by considering walking environment instead of a walking speed scale will be described mainly focusing on differences from Embodiment 1, with referring to Fig. 4 and Fig. 5.

*** Description of Configuration ***

With referring to Fig. 4, a configuration of a hot/cold sensation estimation device 100 will be described.

The hot/cold sensation estimation device 100 includes a walking environment acquisition unit 112 instead of a walking speed scale reception unit 111.

A hot/cold sensation estimation program causes a computer to function as the walking environment acquisition unit 112, not as the walking speed scale reception unit 111.

*** Description of Operation ***

With referring to Fig. 5, a hot/cold sensation estimation method will be described.

In step S021, the walking environment acquisition unit 112 acquires walking environment information.

The walking environment information is environment information of a walking region.

The walking region is the region where walking by the user takes place.

The environment information refers to information of outdoor environment that affects the hot/cold sensation of the user.

For example, the walking environment information indicates temperature, humidity, solar radiation, and so on of Ofuna A-chome, Kamakura city, Kanagawa prefecture.

The walking environment information is acquired as follows.

Walking region information is stored in the storage unit 190 in advance. The walking region information indicates a walking region.

The walking environment acquisition unit 112 sends the walking region information to an environment information server, and receives environment information of the walking region from the environment information server. The received environment information serves as the walking environment information.

The environment information server is a device that provides the environment information.

An instance of the environment information server is a weather forecast server. The weather forecast server is used by the Meteorological Agency, commercial services, and the like, and provides weather information of each region.

The walking environment information may be acquired as follows. An outdoor unit for an air conditioner will be referred to as the outdoor unit.

Various sensors are mounted on the outdoor unit. The various sensors include a temperature sensor and a humidity sensor. Data obtained by the various sensors is referred to as sensor data. The sensor data indicates temperature, humidity, and the like.

The walking environment acquisition unit 112 communicates with the outdoor unit installed in the walking region and receives the sensor data from the various sensors. The received sensor data serves as the walking environment information.

The environment information can be information about indoor environment. Various sensors are installed inside a building.

The walking environment acquisition unit 112 communicates with the various sensors inside the building built in the walking region to receive the sensor data.

The received sensor data serves as the walking environment information.

In step S022, a walking time acquisition unit 120 acquires walking time information.

Step S022 is identical to step S012 in Embodiment 1.

Step S021 and step S022 may be executed in a reverse order.

After step S021 and step S022, the processing proceeds to step S023.

In step S023, a hot/cold sensation estimation unit 130, using the acquired walking environment information and the acquired walking time information as usage information, estimates the hot/cold sensation felt by the user after walking based on the usage information.

Step S023 corresponds to step S013 (refer to Embodiment 1) except that the walking speed scale is replaced with the walking environment information.

After step S023, the processing of the hot/cold sensation estimation method ends.

*** Effects of Embodiment 2 ***

The hot/cold sensation felt after walking depends not only on the walking speed and the walking time, but also on the thermal environment (whether the temperature is high/low and so on) of the place where the user was walking.

Embodiment 2 uses the environment information (temperature, humidity, solar radiation, and so on) of a walking route and walking time to estimate the hot/cold sensation felt after walking.

By using the environment information instead of the walking speed, it becomes possible to estimate more likely hot/cold sensation felt after walking. Moreover, regardless of the device used and no matter who uses which smartphone, it is possible to estimate the hot/cold sensation felt after walking. Furthermore, it is possible to estimate the hot/cold sensation felt after walking while suppressing power consumption of the terminal.

Embodiment 3.

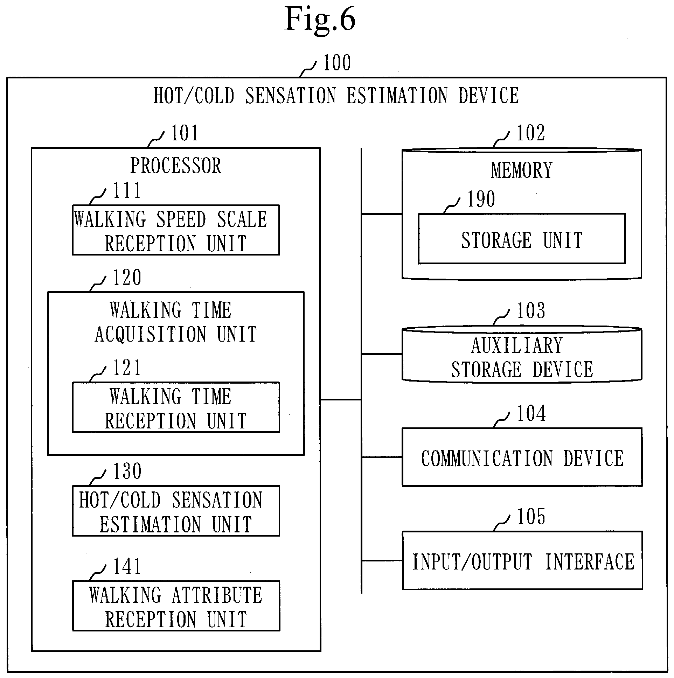

An embodiment for estimating hot/cold sensation felt after walking, considering attributes related to walking of the user, will be described mainly focusing on differences from Embodiment 1, with referring to Fig. 6 through Fig. 9.

*** Description of Configuration ***

With referring to Fig. 6, a configuration of a hot/cold sensation estimation device 100 will be described.

The hot/cold sensation estimation device 100 is further provided with an element which is a walking attribute reception unit 141.

A hot/cold sensation estimation program further causes a computer to function as the walking attribute reception unit 141.

*** Description of Operation ***

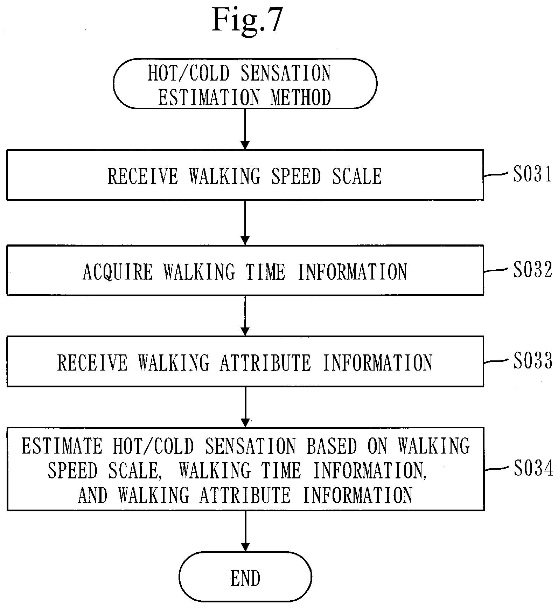

With referring to Fig. 7, a hot/cold sensation estimation method will be described.

In step S031, a walking speed scale reception unit 111 receives a walking speed scale inputted to the hot/cold sensation estimation device 100.

Step S031 is identical to step S011 in Embodiment 1.

In step S032, a walking time acquisition unit 120 acquires walking time information.

Step S032 is identical to step S012 in Embodiment 1.

In step S33, the walking attribute reception unit 141 receives walking attribute information inputted to the hot/cold sensation estimation device 100.

The walking attribute information indicates walking attributes that influence hot/cold sensation of the user.

The walking attributes are attributes related to walking of the user and include user attributes and walking conditions.

Instances of the user attributes include whether the user is heat-sensitive/cold-sensitive, whether the user perspires a lot or not, body fat percentage, age, and so on.

Instances of the walking conditions include whether the walking route is in the shade or sun, whether the user has heavy luggage or not, and so on.

The walking attribute information is inputted using a user interface.

For instance, the walking attribute information is received as follows.

First, the user operates a user terminal after walking to activate the hot/cold sensation estimation device 100.

Next, the walking attribute reception unit 141 displays an attribute input window on a display. The attribute input window has a graphical user interface (GUI) for inputting the walking attribute information.

Then, the user operates the user terminal to input the walking attribute information to the attribute input window.

Then, the walking attribute reception unit 141 receives the inputted walking attribute information.

The order of step S031 to step S033 may be executed in a different order.

After step S031 to step S033, the processing proceeds to step S034.

In step S034, a hot/cold sensation estimation unit 130, using the received walking speed scale, the acquired walking time information, and the received walking attribute information as usage information, estimates the hot/cold sensation felt by the user after walking based on the usage information.

Step S034 corresponds to step S013 (refer to Embodiment 1) except that the walking attribute information is included in the usage information.

After step S034, the processing of the hot/cold sensation estimation method ends.

*** Effects of Embodiment 3 ***

The hot/cold sensation after walking depends not only on the walking speed and the walking time, but also on the walking attribute information (such as whether the user is heat-sensitive/cold-sensitive, body fat percentage, age, and so on) of the user.

Embodiment 3 estimates the hot/cold sensation using also the walking attribute information of the user. This allows a more accurate estimation of the hot/cold sensation after walking.

*** Example of Embodiment 3 ***

Embodiment 3 may be applied to Embodiment 2.

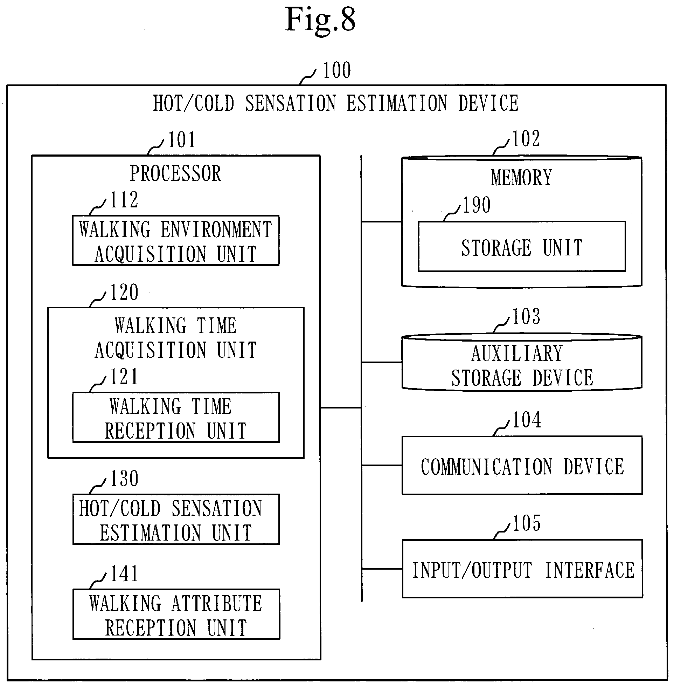

Fig. 8 shows a configuration of a hot/cold sensation estimation device 100 of a case where Embodiment 3 is applied to Embodiment 2.

The hot/cold sensation estimation device 100 is provided with a walking environment acquisition unit 112 instead of a walking speed scale reception unit 111.

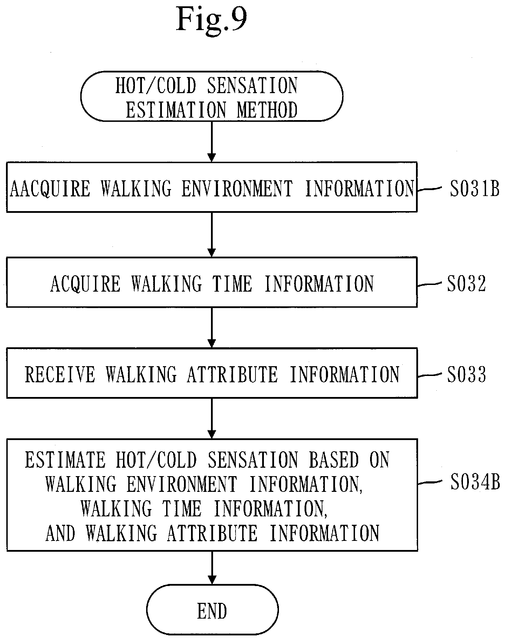

Fig. 9 shows a flowchart of a hot/cold sensation estimation method of the case where Embodiment 3 is applied to Embodiment 2.

In step S031B, the walking environment acquisition unit 112 acquires walking environment information. Step S031B is identical to step S021 of Embodiment 2.

Steps S032 and S033 are as described in Embodiment 3.

In step S034B, a hot/cold sensation estimation unit 130, using acquired walking environment information, acquired walking time information, and received walking attribute information as usage information, estimates hot/cold sensation after walk of a user based on the usage information, . Step S034B corresponds to step S023 (refer to Embodiment 2) except that the walking attribute information is included in the usage information.

Embodiment 4.

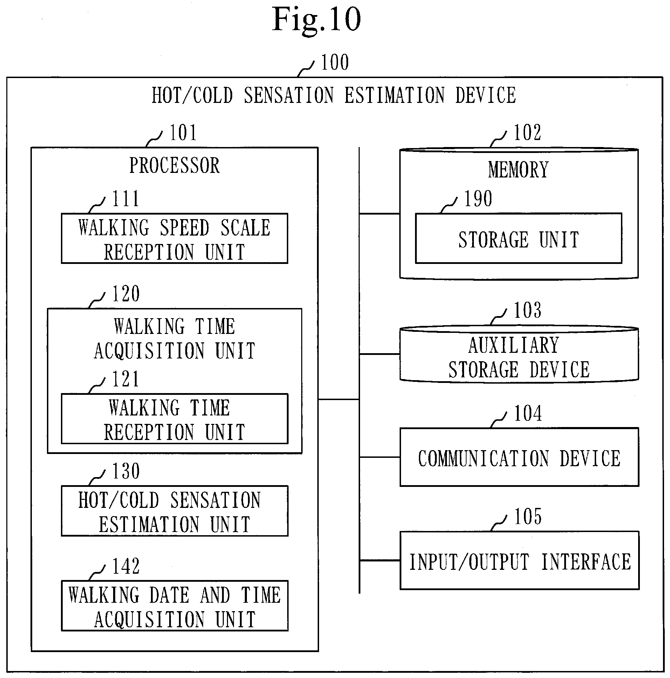

An embodiment for estimating hot/cold sensation after walking, considering walking date and time, will be described mainly focusing on differences from Embodiment 1, with referring to Fig. 10 to Fig. 13.

*** Description of Configuration ***

With referring to Fig. 10, a configuration of a hot/cold sensation estimation device 100 will be described.

The hot/cold sensation estimation device 100 is further provided with an element which is a walking date and time acquisition unit 142.

A hot/cold sensation estimation program further causes a computer to function as the walking date and time acquisition unit 142.

*** Description of Operation ***

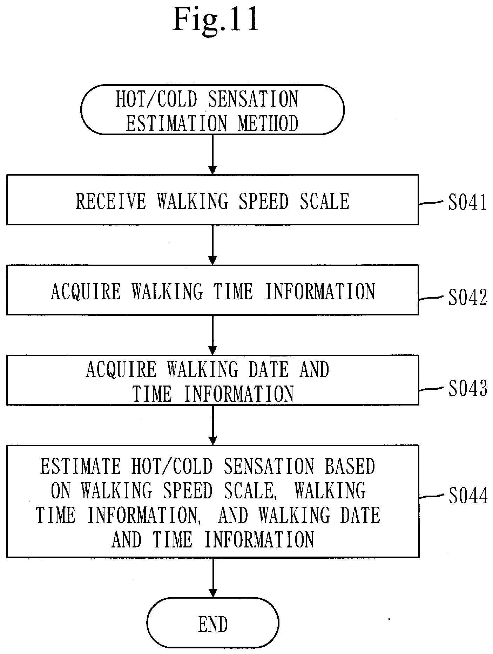

With referring to Fig. 11, a hot/cold sensation estimation method will be described.

In step S041, the walking speed scale reception unit 111 receives a walking speed scale inputted to the hot/cold sensation estimation device 100.

Step S041 is identical to step S011 of Embodiment 1.

In step S042, a walking time acquisition unit 120 acquires walking time information.

Step S042 is identical to step S012 of Embodiment 1.

In step S043, the walking date and time acquisition unit 142 acquires walking date and time information.

The walking date and time information is data indicating the date and time of walking.

The walking date and time corresponds to the date and time when the user was walking.

The walking date and time information is acquired as follows.

The walking date and time acquisition unit 142 acquires current date and time from the OS or the Internet. The acquired data indicating the current date and time serves as the walking date and time information.

Steps S041 to S043 can be executed in a different order.

After steps S041 to S043, the processing proceeds to step S044.

In step S044, a hot/cold sensation estimation unit 130, using received walking speed scale, the acquired walking time information, and the acquired walking date and time information as usage information, estimates the hot/cold sensation after walking of the user based on the usage information.

Step S044 corresponds to step S013 (refer to Embodiment 1) except that the walking date and time information is included in the usage information.

After step S044, the processing of the hot/cold sensation estimation method ends.

*** Effects of Embodiment 4 ***

The hot/cold sensation after walking depends not only on the walking speed and the walking time, but also on the thermal environment of the place where the user was walking (high/low temperature and the like). Date and time corresponds to the thermal environment. For example, the temperature tends to be high at 13:00 in August.

In Embodiment 4, based on an assumption that the date and time corresponds to the thermal environment, an estimation model corresponding to the current date and time information is used. This allows for a more accurate estimation of the hot/cold sensation after walking.

*** Example of Embodiment 4 ***

Embodiment 4 may be applied to Embodiment 2.

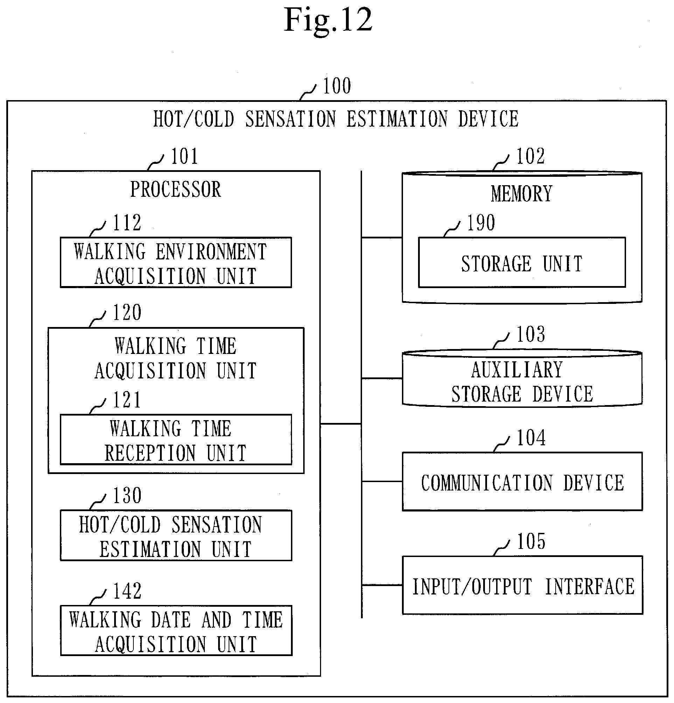

Fig. 12 shows a configuration of a hot/cold sensation estimation device 100 of a case where Embodiment 4 is applied to Embodiment 2.

The hot/cold sensation estimation device 100 is provided with a walking environment acquisition unit 112 instead of a walking speed scale reception unit 111.

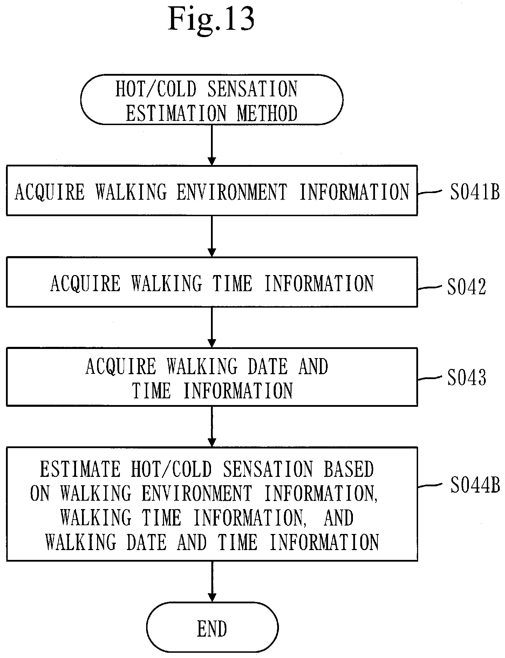

Fig. 13 shows a flowchart of a hot/cold sensation estimation method of the case where Embodiment 4 is applied to Embodiment 2.

In step S041B, the walking environment acquisition unit 112 acquires walking environment information. Step S041B is identical to step S021 of Embodiment 2.

Steps S042 and S043 are as described in Embodiment 3.

In step S044B, a hot/cold sensation estimation unit 130, using the acquired walking environment information, acquired walking time information, and acquired walking date and time information as usage information, estimates the hot/cold sensation after walking of the user based on the usage information. Step S044B corresponds to step S023 (refer to Embodiment 2) except that the walking date and time information is included in the usage information.

Embodiment 5.

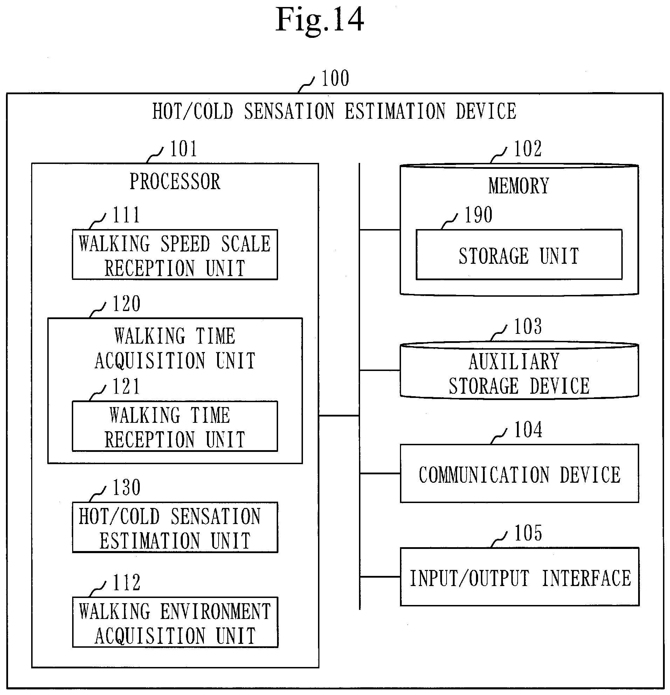

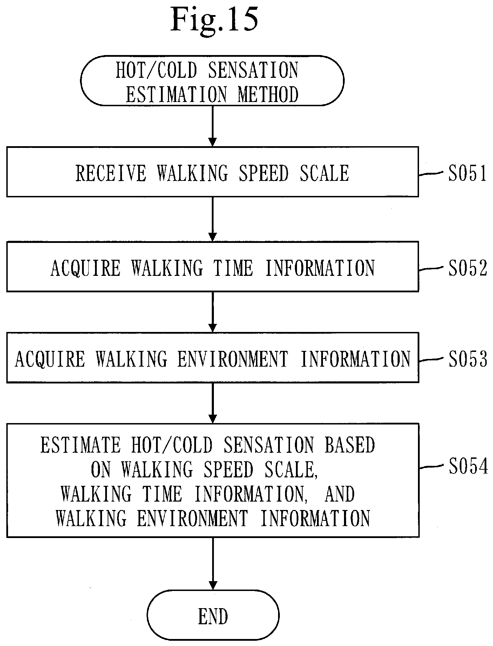

An embodiment for estimating hot/cold sensation after walking, considering walking environment, will be described mainly focusing on differences from Embodiment 1, with referring to Fig. 14 and Fig. 15.

*** Description of Configuration ***

With referring to Fig. 14, a configuration of a hot/cold sensation estimation device 100 will be described.

Furtehrmore, the hot/cold sensation estimation device 100 is provided with an element which is a walking environment acquisition unit 112.

A hot/cold sensation estimation program further causes a computer to function as the walking environment acquisition unit 112.

*** Description of Operation ***

With referring to Fig. 15, a hot/cold sensation estimation method will be described.

In step S051, a walking speed scale reception unit 111 receives a walking speed scale inputted to the hot/cold sensation estimation device 100.

Step S051 is identical to step S011 in Embodiment 1.

In step S052, a walking time acquisition unit 120 acquires walking time information.

Step S052 is identical to step S012 of Embodiment 1.

In step S053, the walking environment acquisition unit 112 acquires walking environment information.

Step S053 is identical to step S021 of Embodiment 2.

Steps S051 to S053 can be executed in a different order.

After steps S051 to S053, the processing proceeds to step S054.

In step S054, a hot/cold sensation estimation unit 130, using the received walking speed scale, the acquired walking time information, and the acquired walking environment information as usage information, estimates hot/cold sensation after walking of the user based on the usage information.

Step S054 corresponds to step S013 (refer to Embodiment 1) except that the walking environment information is included in the usage information.

After step S054, the processing of the hot/cold sensation estimation method ends.

*** Effects of Embodiment 5 ***

The hot/cold sensation after walking depends not only on the walking speed and the walking time, but also on the thermal environment (whether the temperature was high/low and so on) of the place where the user was walking.

Embodiment 5 estimates the hot/cold sensation after walking by using also the environment information (temperature, humidity, sunlight, and so on) of the walking route. This enables a more accurate estimation of the hot/cold sensation after walking.

Embodiment 6.

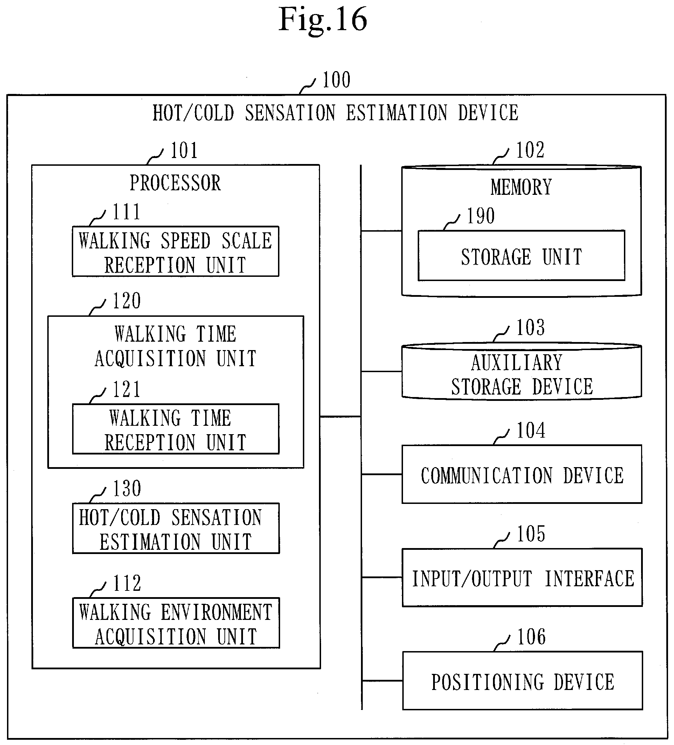

An embodiment for acquiring walking environment information by using a positioning function will be described mainly focusing on differences from Embodiment 5, with referring to Fig. 16 to Fig. 19.

*** Description of Configuration ***

With referring to Fig. 16, a configuration of a hot/cold sensation estimation device 100 will be described.

The hot/cold sensation estimation device 100 is a user terminal possessed by a user while walking.

The hot/cold sensation estimation device 100 is further provided with hardware which is a positioning device 106.

The positioning device 106 has a positioning function to measure a location of the user terminal at each time point. For instance, the positioning device 106 is a receiver of a satellite positioning system (GPS, GNSS, or the like).

*** Description of Operation ***

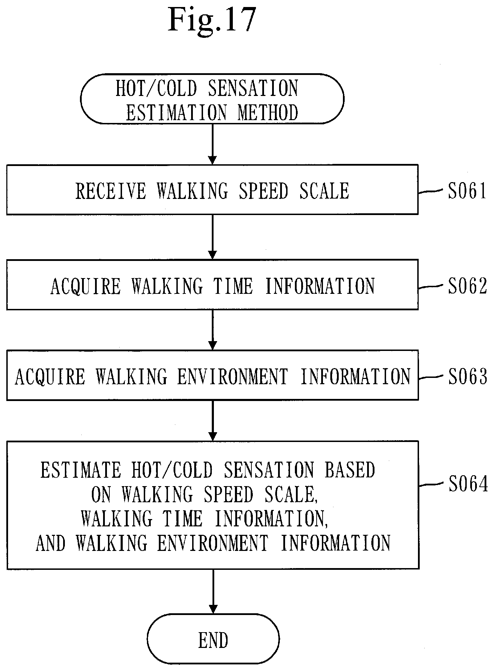

With referring to Fig. 17, a hot/cold sensation estimation method will be described.

In step S061, a walking speed scale reception unit 111 receives a walking speed scale inputted to the hot/cold sensation estimation device 100.

Step S061 is identical to step S011 of Embodiment 1.

In step S062, a walking time acquisition unit 120 acquires walking time information.

Step S062 is identical to step S012 of Embodiment 1.

In step S063, a walking environment acquisition unit 112 acquires walking environment information.

Specifically, the walking environment acquisition unit 112 acquires environment information of a region corresponding to location information of a user terminal. The acquired environment information serves as the walking environment information.

The walking environment information is acquired as follows.

First, the walking environment acquisition unit 112 uses a positioning function of the user terminal to acquire the location information of the user terminal.

Then, the walking environment acquisition unit 112 sends the location information of the user terminal to an environment information server, and receives environment information of the region where the user terminal is located from the environment information server. The received environment information serves as the walking environment information.

Steps S061 to S063 can be executed in a different order.

After steps S061 to S063, the processing proceeds to step S064.

In step S064, a hot/cold sensation estimation unit 130, using the received walking speed scale, the acquired walking time information, and the acquired walking environment information as usage information, estimates the hot/cold sensation after walking of the user based on the usage information.

Step S064 corresponds to step S013 (refer to Embodiment 1) except that the walking environment information is included in the usage information.

After step S064, the processing of the hot/cold sensation estimation method ends.

*** Effects of Embodiment 6 ***

The hot/cold sensation after walking depends not only on the walking speed and the walking time, but also on the thermal environment (high/low temperature and so on) of a place where walking takes place .

Embodiment 6 estimates the hot/cold sensation after walking by using also environment information (temperature, humidity, solar radiation, and so on) of a walking route. This allows to estimate the hot/cold sensation after walking more accurately.

*** Example of Embodiment 6 ***

Embodiment 6 may be applied to Embodiment 2.

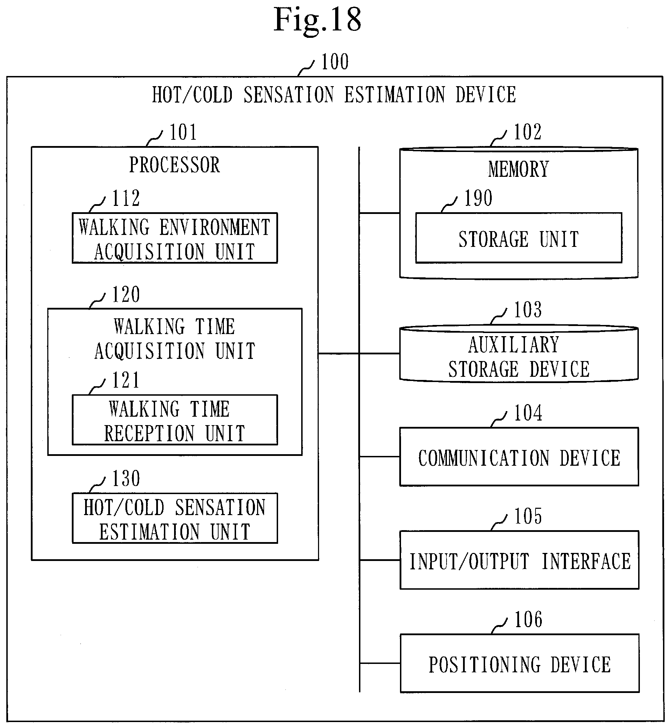

Fig. 18 shows a configuration of a hot/cold sensation estimation device 100 of a case where Embodiment 6 is applied to Embodiment 2.

The hot/cold sensation estimation device 100 is provided with a walking environment acquisition unit 112 instead of a walking speed scale reception unit 111.

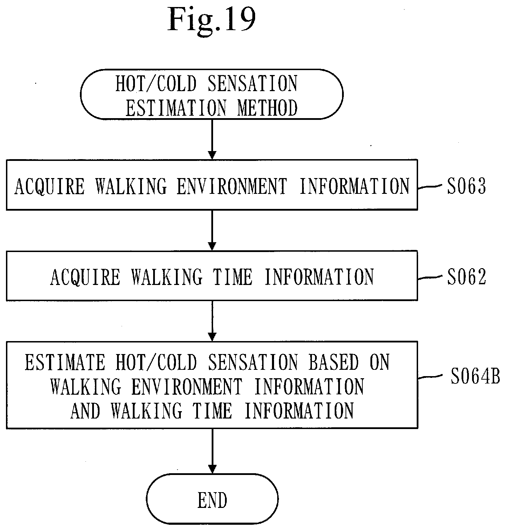

Fig. 19 shows a flowchart of a hot/cold sensation estimation method of the case where Embodiment 6 is applied to Embodiment 2.

Step S063 and step S062 are as described in Embodiment 6.

In step S064B, a hot/cold sensation estimation unit 130, using acquired walking environment information and acquired walking time information as usage information, estimates hot/cold sensation after walking of the user based on the usage information. Step S064B is identical to step S023 of Embodiment 2.

Embodiment 7.

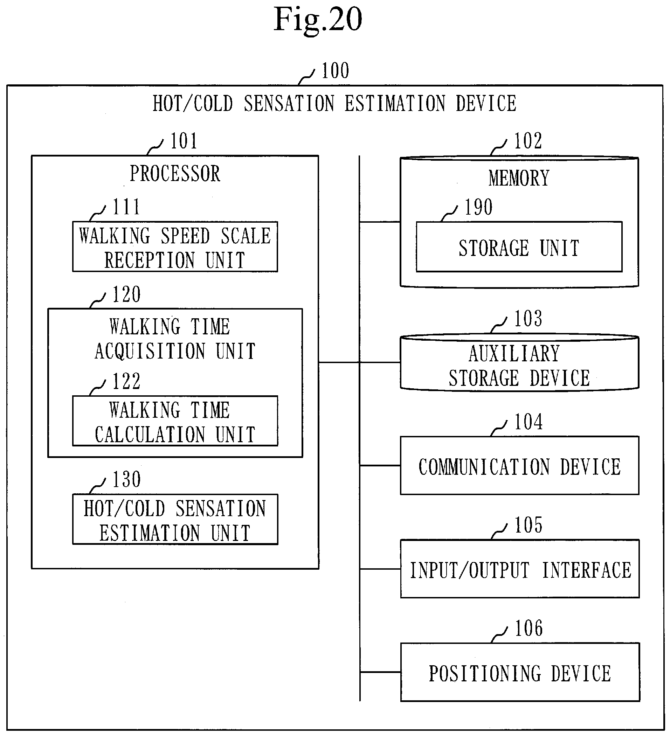

An embodiment for calculating walking time with using location information will be described mainly focusing on differences from Embodiment 1, with referring to Fig. 20 to Fig. 25.

*** Description of Configuration ***

With referring to Fig. 20, a configuration of a hot/cold sensation estimation device 100 will be described.

The hot/cold sensation estimation device 100 is a user terminal possessed by a user during walk of the user.

The hot/cold sensation estimation device 100 is further provided with hardware which is a positioning device 106.

The positioning device 106 has a positioning function for measuring a location of the user terminal at each time point. For instance, the positioning device 106 is a receiver of a satellite positioning system (such as GPS and GNSS).

A walking time acquisition unit 120 is provided with a walking time calculation unit 122 instead of a walking time reception unit 121.

*** Description of Operation ***

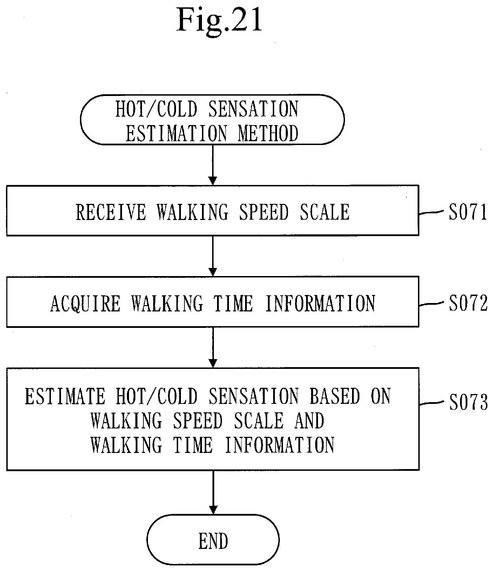

With referring to Fig. 21, a hot/cold sensation estimation method will be described.

In step S071, a walking speed scale reception unit 111 receives a walking speed scale inputted to the hot/cold sensation estimation device 100.

Step S071 is identical to step S011 of Embodiment 1.

In step S072, a walking environment acquisition unit 112 acquires walking time information.

Specifically, the walking time calculation unit 122 calculates walking time as follows. Data indicating the calculated walking time serves as the walking time information.

First, the walking time calculation unit 122 obtains a walking start time point based on walking zone information and location information of the user terminal at each time point.

The walking zone information is data indicating a walking start zone and a walking end zone. The walking zone information is stored in a storage unit 190 in advance.

The walking start zone is a zone that contains a walking start point. The walking start point corresponds to a point where the user starts walking.

The walking end zone is a zone that contains a walking end point. The walking end point corresponds to a point where the user ends walking.

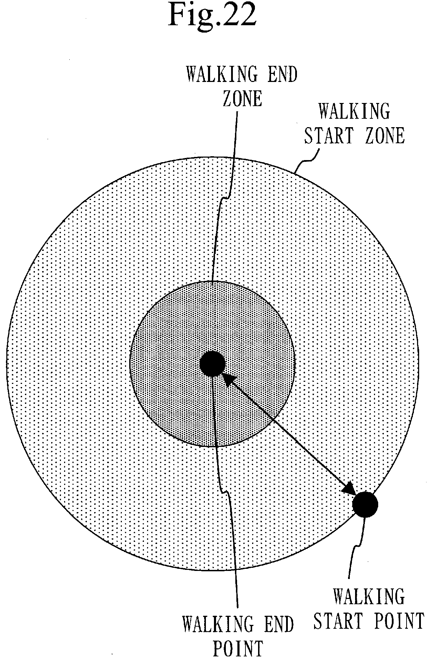

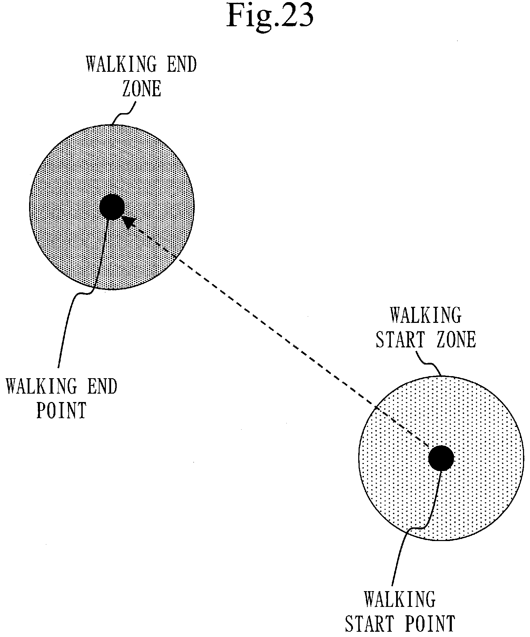

With referring to Fig. 22 and Fig. 23, instances of the walking start zone and walking end zone will be described.

In Fig. 22, the walking start point and the walking end point are set in advance. The walking start zone is expressed as a circle centered on the walking end point, with a distance from the walking end point to the walking start point as a radius. The walking end zone is expressed as a circle centered on the walking end point, with a certain distance as a radius. The certain distance is set in advance.

In Fig. 23, the walking start zone is expressed as a circle centered on the walking start point, with a certain distance as a radius. The walking end zone is expressed as a circle centered on the walking end point, with a certain distance as a radius. The certain distance is set in advance.

However, the walking start zone and the walking end zone may each be a zone with a shape other than a circle. For instance, the walking start zone and the walking end zone may each be a rectangular zone. Also, the walking start zone and the walking end zone may be zones with shapes that differ from each other.

Returning to Fig. 21, the explanation of step S072 will be continued.

The location information of the user terminal at each time point is acquired using the positioning function of the user terminal.

The walking start time point is the time point when the user terminal has entered the walking start zone.

Next, the walking time calculation unit 122 obtains a walking end time point based on the walking zone information and the location information of the user terminal at each time point.

The walking end time point is the time point when the user terminal has entered the walking end zone.

Then, the walking time calculation unit 122 calculates the walking time using the walking start time point and the walking end time point.

Specifically, the walking time calculation unit 122 calculates a duration of time from the walking start time point (t1) to the walking end time point (t2). The calculated time duration serves as the walking time (Δt).

The walking time (Δt) is calculated by \executing the following formula.

$$\Delta t = t2 - t1$$

The walking time calculation unit 122 may calculate the walking time (Δt) by subtracting a walking stop time (t3). The walking stop time (t3) is a duration of time during which walking stopped between the walking start time point and the walking end time point. For instance, a period of time where the location of the user terminal has not changed for a certain time is considered as the time period where walking stopped. Note that if the location of the user terminal has not changed by a certain distance, it is considered that the location of the user terminal has not changed.

The walking time (Δt) is calculated by executing the following formula.

$$\Delta t = t2 - t1 - t3$$

Step S071 and step S072 may be executed in a reverse order.

After step S071 and step S072, the processing proceeds to step S073.

In step S073, the hot/cold sensation estimation unit 130, using the received walking speed scale and the acquired walking time information as usage information, estimates hot/cold sensation after walking of the user based on the usage information.

Step S073 is identical to step S013 of Embodiment 1.

After step S073, the processing of the hot/cold sensation estimation method ends.

*** Effects of Embodiment 7 ***

The walking time may vary from day to day. In order to accurately estimate the hot/cold sensation, it is necessary to acquire the walking time each time.

Embodiment 7 calculates the walking time automatically using location information. This can eliminate time and effort of the user required for inputting.

*** Example of Embodiment 7 ***

Embodiment 7 may be applied to Embodiment 2.

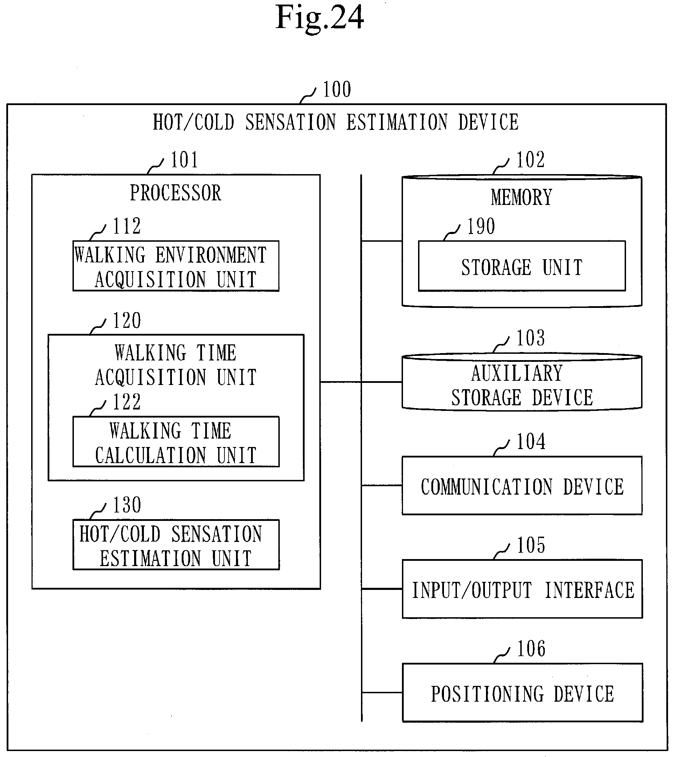

Fig. 24 shows a configuration of a hot/cold sensation estimation device 100 of a case where Embodiment 7 is applied to Embodiment 2.

The hot/cold sensation estimation device 100 is provided with a walking environment acquisition unit 112 instead of a walking speed scale reception unit 111.

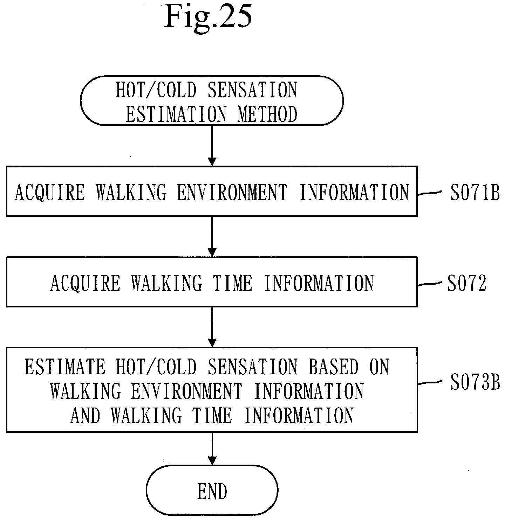

Fig. 25 shows a flowchart of a hot/cold sensation estimation method of the case where Embodiment 7 is applied to Embodiment 2.

In step S071B, the walking environment acquisition unit 112 acquires walking environment information. Step S071B is identical to step S021 of Embodiment 2.

Step S072 is as described in Embodiment 7.

In step S073B, the hot/cold sensation estimation unit 130, using the acquired walking environment information and acquired walking time information as usage information, estimates the hot/cold sensation after walking of the user based on the usage information. Step S073B is identical to step S023 of Embodiment 2.

Embodiment 8.

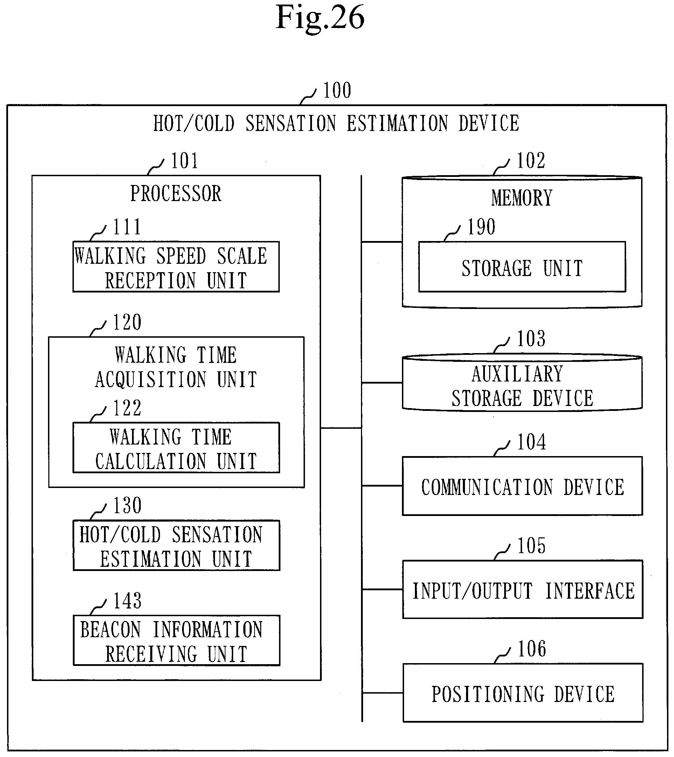

An embodiment for calculating walking time using location information and beacon information will be described mainly focusing on differences from Embodiment 7, with referring to Fig. 26 through Fig. 30.

*** Description of Configuration ***

With referring to Fig. 26, a configuration of a hot/cold sensation estimation device 100 will be described.

The hot/cold sensation estimation device 100 is further provided with an element which is a beacon information receiving unit 143.

A hot/cold sensation estimation program further causes a computer to function as the beacon information receiving unit 143.

*** Description of Operation ***

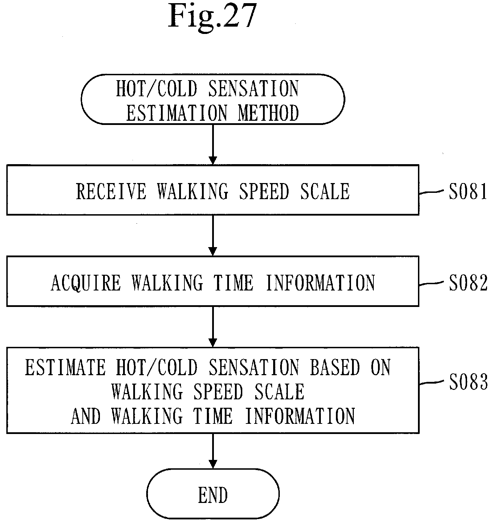

With referring to Fig. 27, a hot/cold sensation estimation method will be described.

In step S081, a walking speed scale reception unit 111 receives a walking speed scale inputted to the hot/cold sensation estimation device 100.

Step S081 is identical to step S011 of Embodiment 1.

In step S082, a walking environment acquisition unit 112 acquires walking time information.

Specifically, a walking time calculation unit 122 calculates a walking time as follows. Data indicating the calculated walking time serves as the walking time information.

One or more walking end zones are set, and each walking end zone is provided with a beacon. For example, the beacon is installed inside a building that is a walking destination of the user.

The beacon emits beacon information. The beacon information is data containing an individual identifier. The individual identifier identifies one beacon. For instance, an individual identification number is used as the individual identifier.

If a user terminal enters a communication zone of any beacon, the beacon information receiving unit 143 receives beacon information transmitted from that beacon. A communication zone of the beacon is a zone within a reachable range of reach of the beacon information.

First, the walking time calculation unit 122 obtains a walking start time point based on walking zone information and location information of the user terminal at each time point. The walking zone information indicates a walking start zone.

Subsequently, the walking time calculation unit 122 obtains a time point at which the beacon information was received. The obtained time point serves as a walking end time point.

Then, the walking time calculation unit 122 calculates the walking time using the walking start time point and the walking end time point.

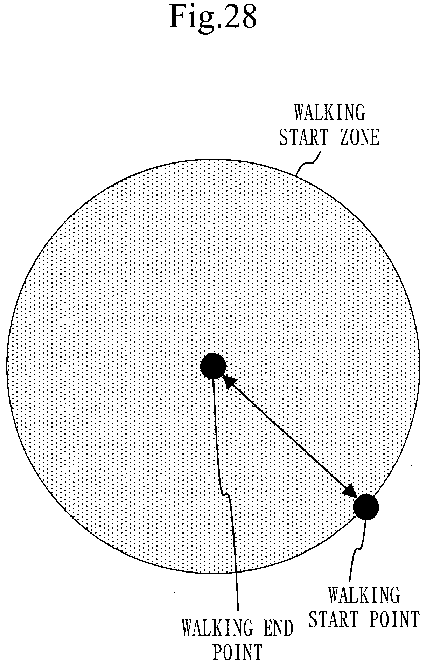

With referring to Fig. 28, an instance of the walking start zone will be described.

A walking start point is determined in advance.

A walking end point is a location of a beacon identified by an individual identifier indicated in the received beacon information.

The walking start zone is expressed as a circle centered on the walking end point, with a distance from the walking end point to the walking start point as a radius.

The walking start zone is calculated as follows.

A list of beacons is stored in a storage unit 190 in advance. The list of beacons shows, for each beacon, the individual identifier and the location information associated to each other.

First, the walking time calculation unit 122 acquires location information associated with an individual identifier identical to the individual identifier indicated in the received beacon information, from the list of beacons.

Then, the walking time calculation unit 122, using a location indicated by the acquired location information as the walking end point, calculates the walking start zone.

Returning to Fig. 27, the explanation will be continued.

Step S081 and step S082 can be executed in a reverse order.

After step S081 and step S082, the processing proceeds to step S083.

In step S083, a hot/cold sensation estimation unit 130, using a received walking speed scale and acquired walking time information as usage information, estimates the hot/cold sensation after walking of the user based on the usage information.

Step S083 is identical to step S013 of Embodiment 1.

After step S083, the processing of the hot/cold sensation estimation method ends.

*** Effects of Embodiment 8 ***

Embodiment 8 uses location information and beacon information to automatically calculate walking time. This can reduce time and effort of the user required for inputting. Additionally, the walking time can be calculated more accurately.

*** Example of Embodiment 8 ***

Embodiment 8 may be applied to Embodiment 2.

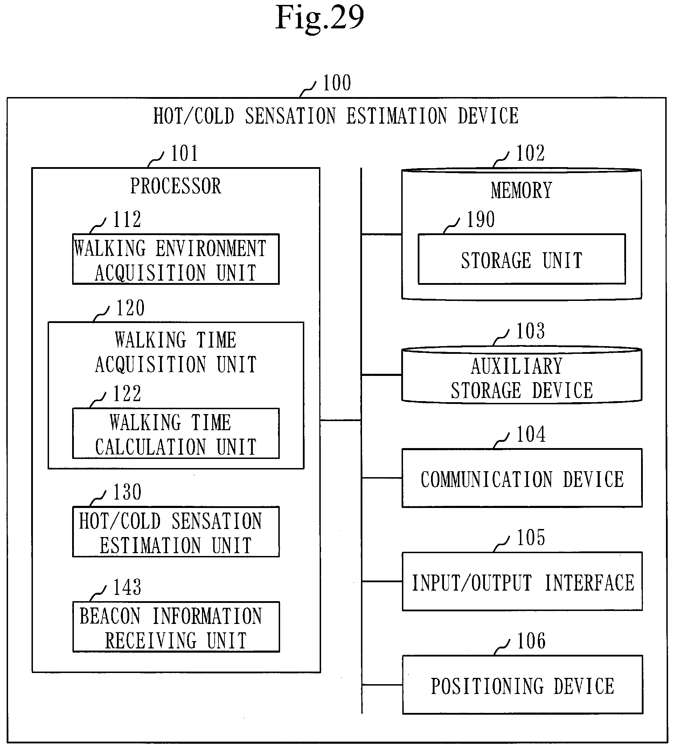

Fig. 29 shows a configuration of a hot/cold sensation estimation device 100 of a case where Embodiment 8 is applied to Embodiment 2.

The hot/cold sensation estimation device 100 is provided with a walking environment acquisition unit 112 instead of a walking speed scale reception unit 111.

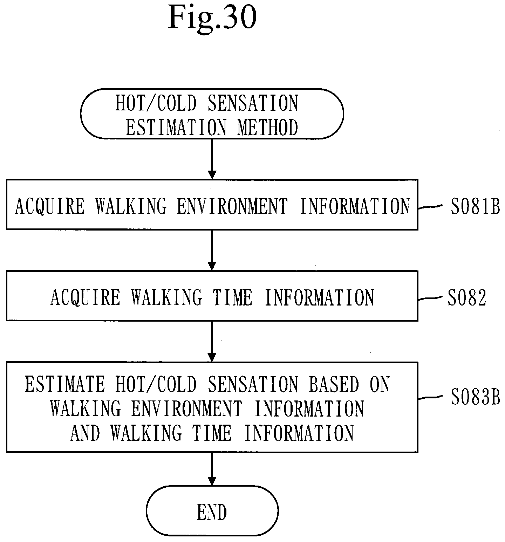

Fig. 30 shows a flowchart of a hot/cold sensation estimation method of the case where Embodiment 8 is applied to Embodiment 2.

In step S081B, the walking environment acquisition unit 112 acquires walking environment information. Step S081B is identical to step S021 of Embodiment 2.

Step S082 is as described in Embodiment 8.

In step S083B, a hot/cold sensation estimation unit 130, using the acquired walking environment information and acquired walking time information as usage information, estimates hot/cold sensation after walking of the user based on the usage information. Step S083B is identical to step S023 of Embodiment 2.

Embodiment 9.

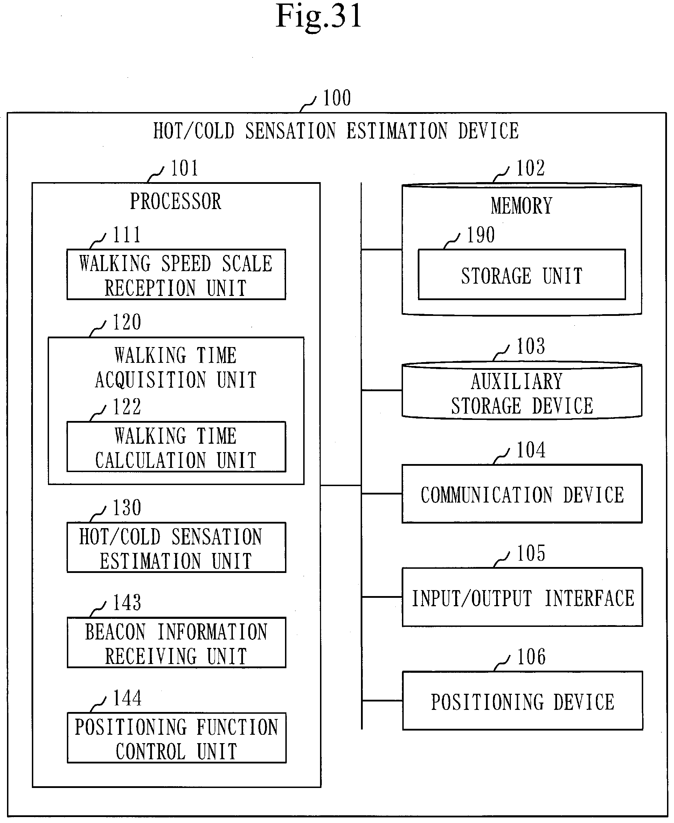

An embodiment for suppressing power consumption of a user terminal will be described mainly focusing on differences from Embodiment 8, with referring to Fig. 31 to Fig. 34.

*** Description of Configuration ***

With referring to Fig. 31, a configuration of a hot/cold sensation estimation device 100 will be described.

The hot/cold sensation estimation device 100 is further provided with an element which is a positioning function control unit 144.

A hot/cold sensation estimation program further causes a computer to function as the positioning function control unit 144.

*** Description of Operation ***

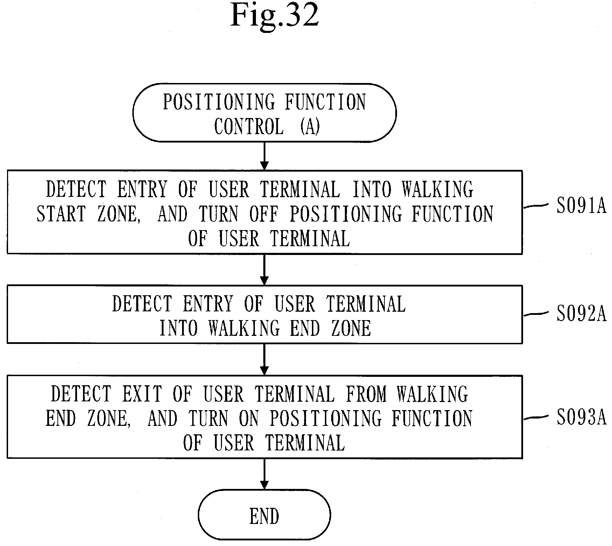

With referring to Fig. 32, a positioning function control (A) will be described.

The positioning function control (A) is an instance of positioning function control.

The positioning function control is a process included in a hot/cold sensation estimation method and is executed by the positioning function control unit 144.

In step S091A, the positioning function control unit 144 detects that the user terminal has entered a walking start zone.

The detection is executed using walking zone information and positional information of the user terminal at each time point.

The detection could also be executed by a walking time calculation unit 122.

Then, the positioning function control unit 144 turns off a positioning function of the user terminal. In other words, the positioning function control unit 144 turns off a communication device 104.

In step S092A, the positioning function control unit 144 detects that the user terminal has entered a walking end zone. In other words, the positioning function control unit 144 detects that the user terminal has approached a walking end point.

Specifically, the positioning function control unit 144 detects that beacon information is received. The positioning function control unit 144 may detect that a radio wave intensity of the beacon information at the time of reception is equal to or above a threshold.

In step S093A, the positioning function control unit 144 detects that the user terminal has exited the walking end zone.

Specifically, the positioning function control unit 144 detects that the beacon information is no longer received. The positioning function control unit 144 may detect that a radio wave intensity of the beacon information at the time of reception is less than the threshold.

Then, the positioning function control unit 144 turns on the positioning function of the user terminal. In other words, the positioning function control unit 144 turns on the communication device 104.

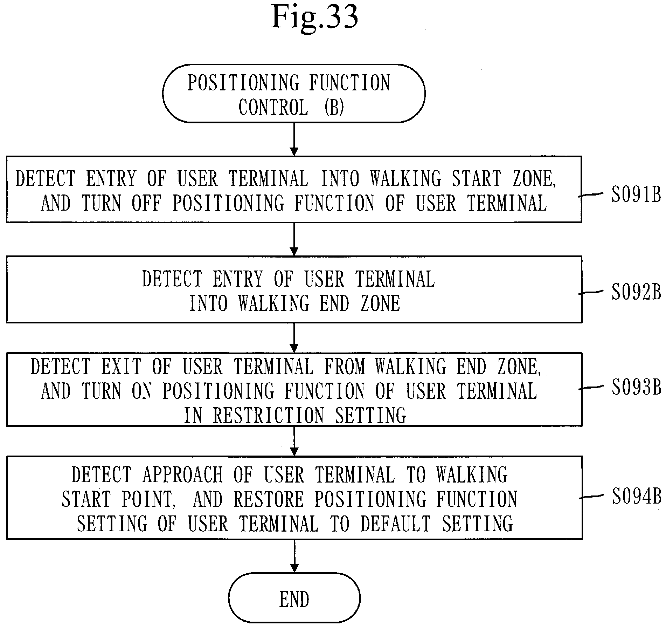

With referring to Fig. 33, the positioning function control (B) will be described.

The positioning function control (B) is an instance of positioning function control.

In step S091B, the positioning function control unit 144 detects that the user terminal has entered the walking start zone.

Then, the positioning function control unit 144 turns off the positioning function of the user terminal.

Step S091B is identical to step S091A.

In step S092B, the positioning function control unit 144 detects that the user terminal has entered the walking end zone.

Step S092B is identical to step S092A.

In step S093B, the positioning function control unit 144 detects that the user terminal has exited the walking end zone. The detection method is the same as the method used in step S093A.

Then, the positioning function control unit 144 turns on the positioning function of the user terminal in restriction setting. In other words, the positioning function control unit 144 turns on the communication device 104 in the restriction setting.

The restriction setting is setting to suppress power consumption of the positioning function. For instance, in the restriction setting, at least either a positioning frequency or a positioning accuracy is lower than in default setting.

In step S094B, the positioning function control unit 144 detects that the user terminal has approached a walking start point.

Specifically, the positioning function control unit 144 detects that a distance from the user terminal to the walking start point has become less than or equal to a certain distance.

Then, the positioning function control unit 144 restores the positioning function setting of the user terminal to the default setting. In other words, the positioning function control unit 144 restores the setting of the communication device 104 to the default setting.

The default setting is the setting employed before the positioning function is turned off (normal state).

*** Effects of Embodiment 9 ***

Use of the positioning function of a smartphone consumes power.

Embodiment 9 switches parameters such as the on/off state of the positioning function, positioning frequency, and positioning accuracy according to location information. By doing this, it is possible to reduce the power consumption of the smartphone while saving time and effort of the user required for inputting.

*** Example of Embodiment 9 ***

Embodiment 9 can also be applied to Embodiment 2.

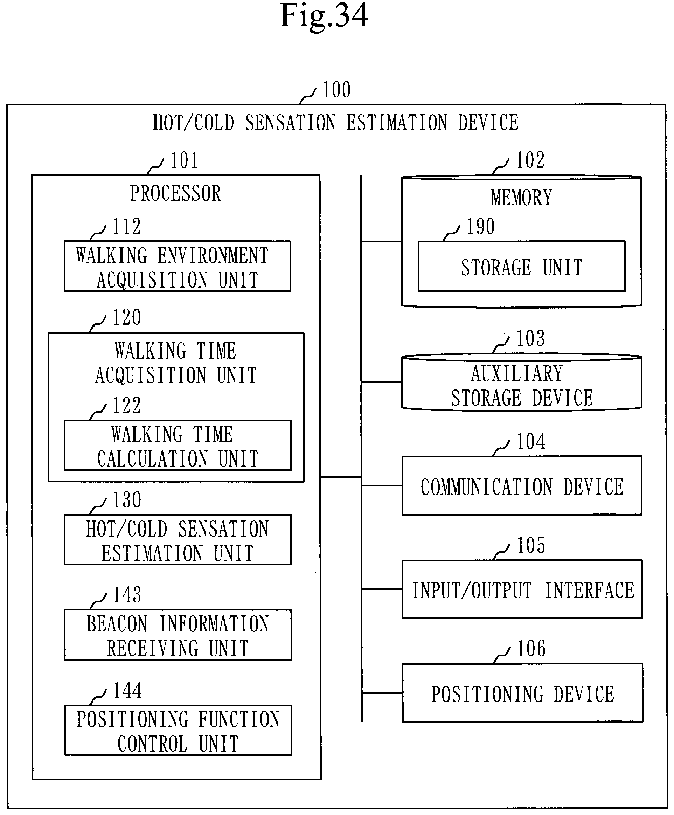

Fig. 34 shows a configuration of a hot/cold sensation estimation device 100 of a case where Embodiment 9 is applied to Embodiment 2.

The hot/cold sensation estimation device 100 is provided with a walking environment acquisition unit 112 instead of a walking speed scale reception unit 111.

Operations of the positioning function control unit 144 are as described in Embodiment 9.

Embodiment 10.

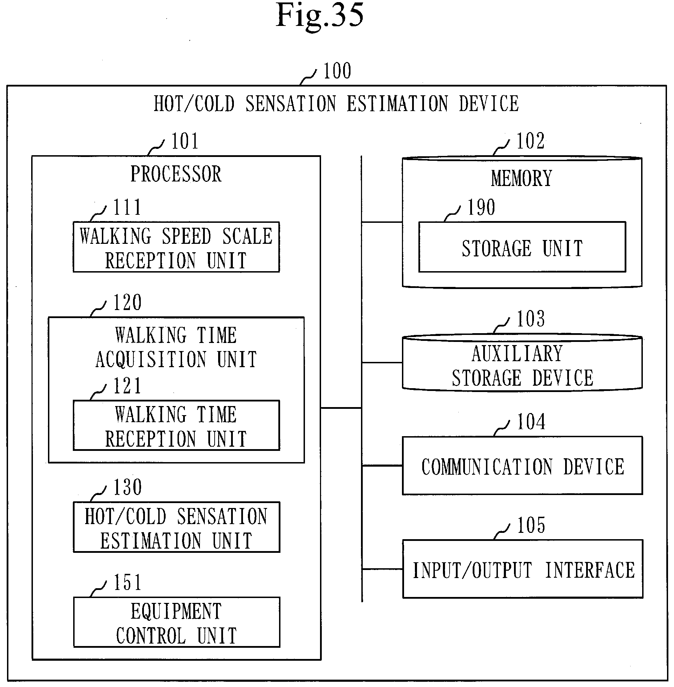

An embodiment for controlling an air conditioner and the like in accordance with estimated hot/cold sensation will be described mainly focusing on differences from Embodiment 1, with referring to Fig. 35 through Fig. 38.

*** Description of Configuration ***

With referring to Fig. 35, a configuration of a hot/cold sensation estimation device 100 will be described.
The hot/cold sensation estimation device 100 is further provided with an element which is an equipment control unit 151.
A hot/cold sensation estimation program further causes a computer to function as the equipment control unit 151.

*** Description of Operation ***

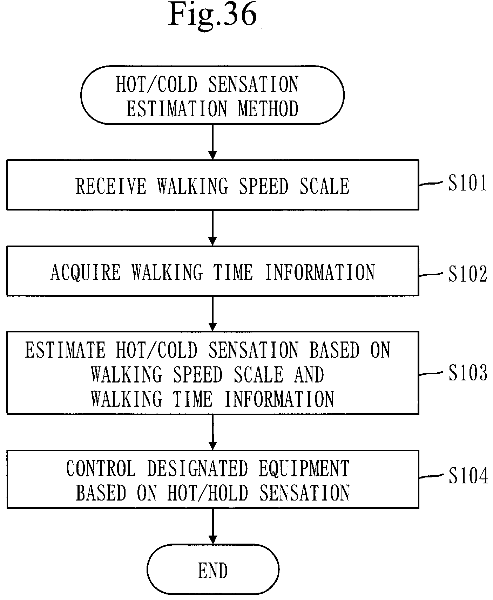

With referring to Fig. 36, a hot/cold sensation estimation method will be described.
In step S101, a walking speed scale reception unit 111 receives a walking speed scale inputted to the hot/cold sensation estimation device 100.
Step S101 is identical to step S011 in Embodiment 1.
In step S102, a walking environment acquisition unit 112 acquires walking time information.
Step S102 is identical to step S012 of Embodiment 1.
Step S101 and step S102 can be executed in a reverse order.
After step S101 and step S102, the processing proceeds to step S103.
In step S103, a hot/cold sensation estimation unit 130, using the received walking speed scale and the acquired walking time information as usage information, estimates hot/cold sensation after walking of a user based on the usage information.
Step S103 is identical to step S013 in Embodiment 1.
In step S104, the equipment control unit 151 controls designated equipment based on the estimated hot/cold sensation.
The designated equipment is designated heating and cooling equipment. The specified equipment is registered in advance.
The heating and cooling equipment is equipment that affects human hot/cold sensation.
Instances of the heating and cooling equipment include an air conditioner, an electric fan, a fan, a ventilation device, and lighting equipment.
Specifically, the equipment control unit 151 adjusts settings of the designated equipment based on the estimated hot/cold sensation and favorite setting of the user for the designated equipment.
The designated equipment is controlled as follows.

Favorite setting information is stored in a storage unit 190 in advance. The favorite setting information is data indicating favorite setting of the user for the designated equipment.
First, the equipment control unit 151 determines the setting of the designated equipment based on the estimated hot/cold sensation and the favorite setting which is indicated in the favorite setting information.
Then, the equipment control unit 151 communicates with the designated equipment to change the setting of the designated equipment to the determined setting.
For instance, an air conditioner which is specified equipment is controlled as follows.
The favorite setting information indicates a favorite temperature.
If the estimated hot/cold sensation is a value of +2 or higher, the equipment control unit 151 sets the air volume to "strong" and the air direction to "swing". By setting the air direction to "swing", the wind will hit the user. As it is assumed that a state of high hot/cold sensation only lasts for a few minutes, the equipment control unit 151, after a certain amount of time has passed, may change the air volume to "weak" and the air direction to "horizontal". By setting the air direction to "horizontal", the wind will not hit the user.
The equipment control unit 151 sets the temperature to the favorite temperature. The equipment control unit 151 may adjust the favorite temperature based on the estimated hot/cold sensation to set the temperature to an adjusted favorite temperature. As it is assumed that the state of high hot/cold sensation only lasts for a few minutes, the equipment control unit 151, after a certain amount of time has passed, may change the temperature back to the pre-adjustment original favorite temperature.
For instance, an air conditioner, which is designated equipment, is controlled as follows.
The favorite setting information indicates a favorite air volume and a favorite wind direction.
If the estimated hot/cold sensation is a value of +2 or higher, the equipment control unit 151 sets the air volume to "strong" and the air direction to "swing". As it is assumed that a state of high hot/cold sensation only lasts for a few minutes, the equipment control unit 151, after a certain amount of time has passed, changes the air volume setting to the favorite air volume and the wind direction setting to the favorite wind direction.
For example, if the specified equipment type is a fan or a ventilation device, and the estimated hot/cold sensation is high, the equipment control unit 151 increases the air volume.
For instance, if the specified equipment type is lighting equipment and the estimated hot/cold sensation is high, the equipment control unit 151 lowers the color temperature of the lighting.
After step S104, the processing of the hot/cold sensation estimation method ends.

*** Effects of Embodiment 10 ***

Embodiment 10 automatically changes the setting of the designated air conditioner based on the estimated hot/cold sensation. This can realize comfortable air conditioning control based on the hot/cold sensation after walking, using the designated air conditioner.

*** Example of Embodiment 10 ***

Embodiment 10 may be applied to Embodiment 2.

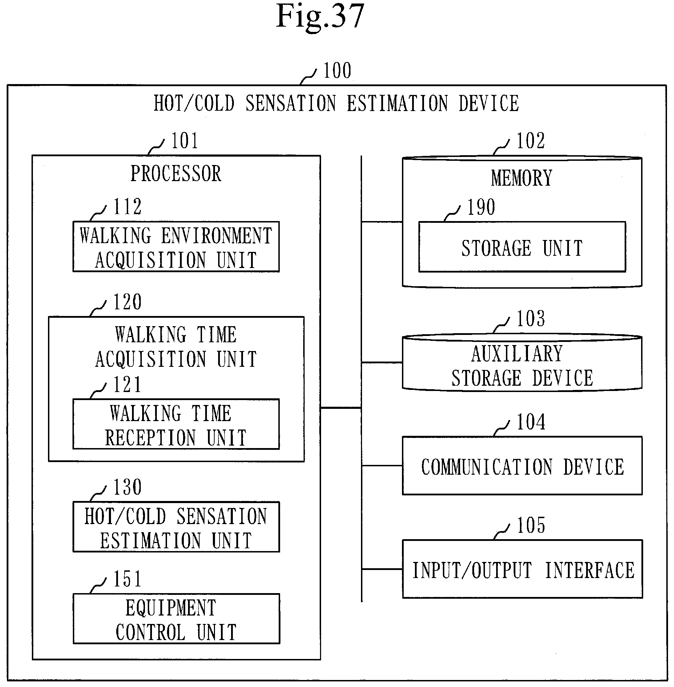

Fig. 37 shows a configuration of a hot/cold sensation estimation device 100 of a case where Embodiment 10 is applied to Embodiment 2.

The hot/cold sensation estimation device 100 is provided with a walking environment acquisition unit 112 instead of a walking speed scale reception unit 111.

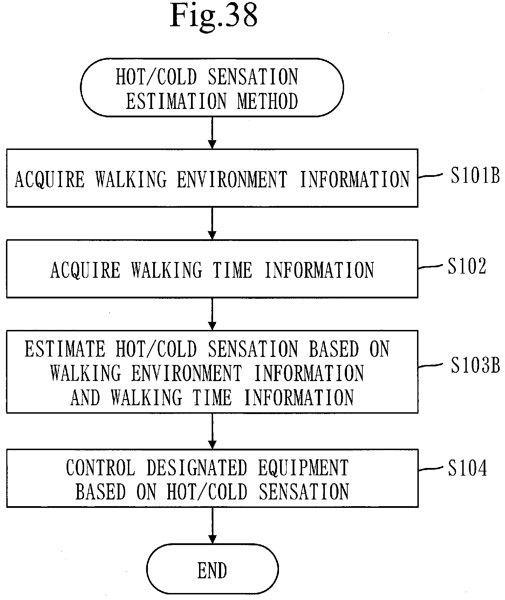

Fig. 38 shows a flowchart of a hot/cold sensation estimation method of the case where Embodiment 10 is applied to Embodiment 2.

In step S101B, the walking environment acquisition unit 112 acquires walking environment information. Step S101B is identical to step S021 of Embodiment 2.

Step S102 is as described in Embodiment 10.

In step S103B, a hot/cold sensation estimation unit 130, using the acquired walking environment information and acquired walking time information as usage information, estimates hot/cold sensation after walking of a user based on the usage information. Step S103B is identical to step S023 of Embodiment 2.

Step S104 is as described in Embodiment 10.

Embodiment 11.

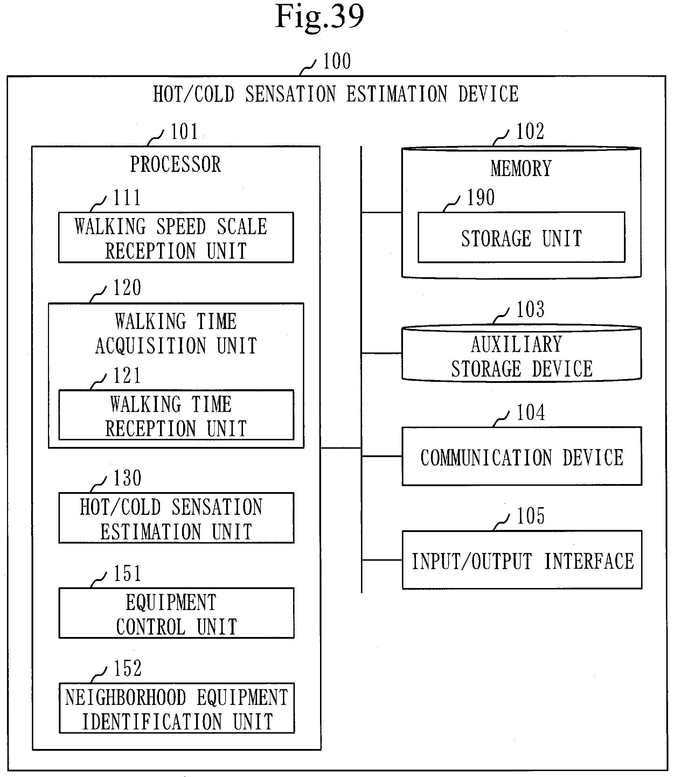

An embodiment for controlling an air conditioner and the like in accordance with estimated hot/cold sensation will be described mainly focusing on differences from Embodiment 10, with referring to Fig. 39 to Fig. 42.

*** Description of Configuration ***

With referring to Fig. 39, a configuration of a hot/cold sensation estimation device 100 will be described.

The hot/cold sensation estimation device 100 is further provided with an element which is a neighborhood equipment identification unit 152.

A hot/cold sensation estimation program further causes a computer to function as the neighborhood equipment identification unit 152.

*** Description of Operation ***

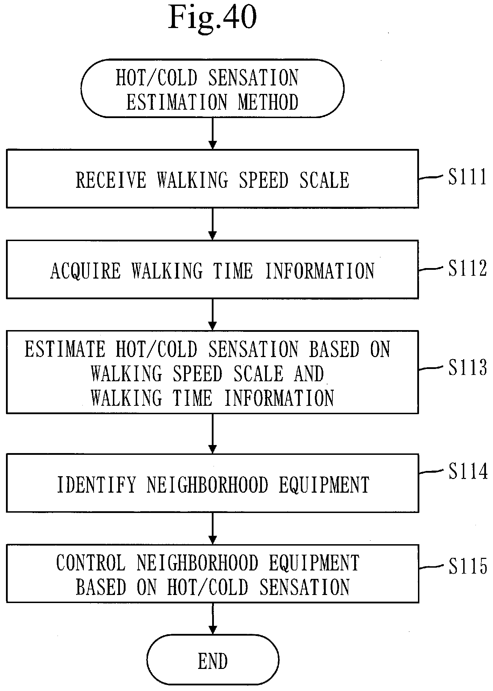

With referring to Fig. 40, a hot/cold sensation estimation method will be described.

In step S111, a walking speed scale reception unit 111 receives a walking speed scale inputted to the hot/cold sensation estimation device 100.

Step S111 is identical to step S011 of Embodiment 1.

In step S112, a walking environment acquisition unit 112 acquires walking time information.

Step S112 is identical to step S012 of Embodiment 1.

Step S111 and step S112 can be executed in a reverse order.

After step S111 and step S112, the processing proceeds to step S113.

In step S113, a hot/cold sensation estimation unit 130, using the received walking speed scale and the acquired walking time information as the usage information, estimates hot/cold sensation after walking of a user based on the usage information.

Step S113 is identical to step S013 of Embodiment 1.

In step S114, the neighborhood equipment identification unit 152 receives beacon information from at least one of a plurality of pieces of heating/cooling equipment.

The plurality of pieces of heating/cooling equipment are located at positions different from each other.

Each heating/cooling equipment has a beacon.

The beacon transmits beacon information. The beacon information is data containing an individual identifier. The individual identifier identifies one beacon. For example, an individual identification number is used as the individual identifier.

Then, the neighborhood equipment identification unit 152 selects one of the plurality of pieces of heating/cooling equipment based on a reception result of the beacon information. The selected heating/cooling equipment will be referred to as neighborhood equipment.

The neighborhood equipment is heating/cooling equipment provided near the user who is after walking.

The neighborhood equipment is identified as follows.

A list of heating/cooling equipment is stored in a storage unit 190 in advance. The list of heating/cooling equipment shows, for each heating/cooling equipment, an equipment identifier and a beacon identifier associated to each other.

When beacon information is received from one heating/cooling equipment, the neighborhood equipment identification unit 152 selects an equipment identifier associated with a beacon identifier identical to an individual identifier indicated by the received beacon information, from the list of heating/cooling equipment. The heating/cooling equipment identified by the selected equipment identifier serves as the neighborhood equipment.

When beacon information is received from two or more pieces of heating/cooling equipment, the neighborhood equipment identification unit 152 selects an equipment identifier associated with a beacon identifier identical to an individual identifier indicated in beacon information with the strongest radio wave intensity at the time of reception or with the shortest radio wave reach distance, from the list of heating/cooling equipment. The heating/cooling equipment identified by the selected equipment identifier serves as the neighborhood equipment.

When Bluetooth (registered trademark) is used for beacon information communication, the radio wave intensity is utilized.

When wireless communication called UWB is used for beacon information communication, radio wave reach distance is utilized. Note that UWB is short for Ultra Wide Band.

Step S114 may be executed before step S113.

In step S115, an equipment control unit 151 controls the neighborhood equipment based on estimated hot/cold sensation.

A method of controlling the neighborhood equipment is the same as the method of controlling the designated equipment in step S104 of Embodiment 10.

After step S115, the processing of the hot/cold sensation estimation method ends.

*** Effects of Embodiment 11 ***

Embodiment 11 automatically changes the setting of the neighborhood air conditioner based on the estimated hot/cold sensation. This can realize comfortable air conditioning control based on the hot/cold sensation after walking, using the neighborhood air conditioner.

*** Example of Embodiment 11 ***

Embodiment 11 may be applied to Embodiment 2.

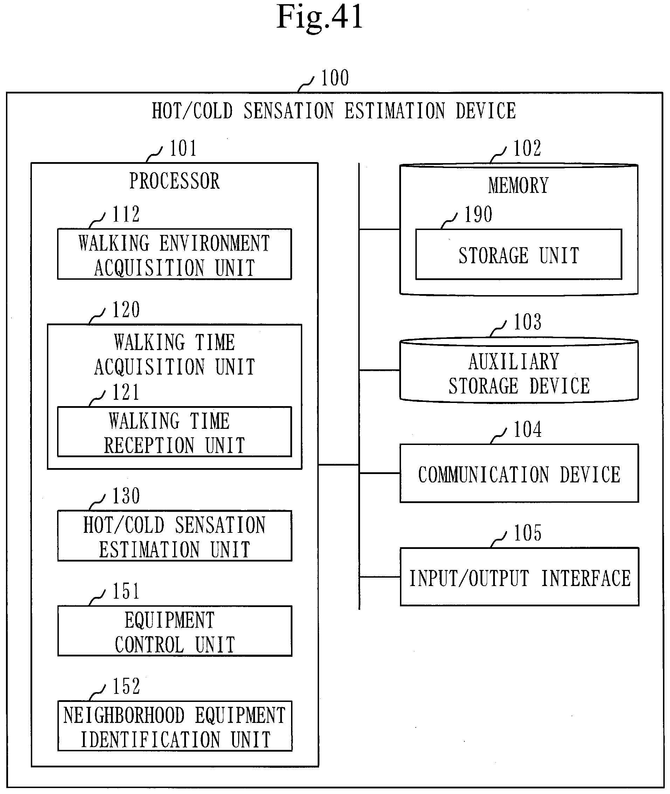

Fig. 41 shows a configuration of a hot/cold sensation estimation device 100 of a case where Embodiment 11 is applied to Embodiment 2.

The hot/cold sensation estimation device 100 is provided with a walking environment acquisition unit 112 instead of a walking speed scale reception unit 111.

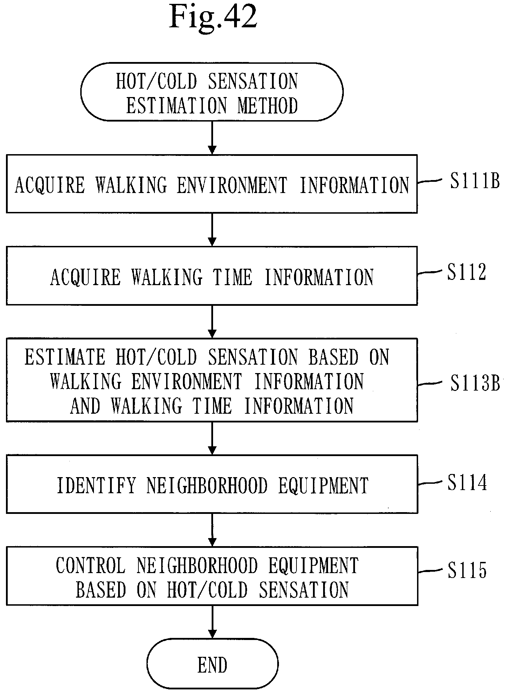

Fig. 42 shows a flowchart of a hot/cold sensation estimation method of the case where Embodiment 11 is applied to Embodiment 2.

In step S111B, the walking environment acquisition unit 112 acquires walking environment information. Step S111B is identical to step S021 of Embodiment 2.

Step S112 is as described in Embodiment 10.

In step S113B, a hot/cold sensation estimation unit 130, using the acquired walking environment information and acquired walking time information as usage information, estimates hot/cold sensation after walking of the user based on the usage information. Step S113B is identical to step S023 of Embodiment 2.

Steps S114 and S115 are as described in Embodiment 11.

Embodiment 12.

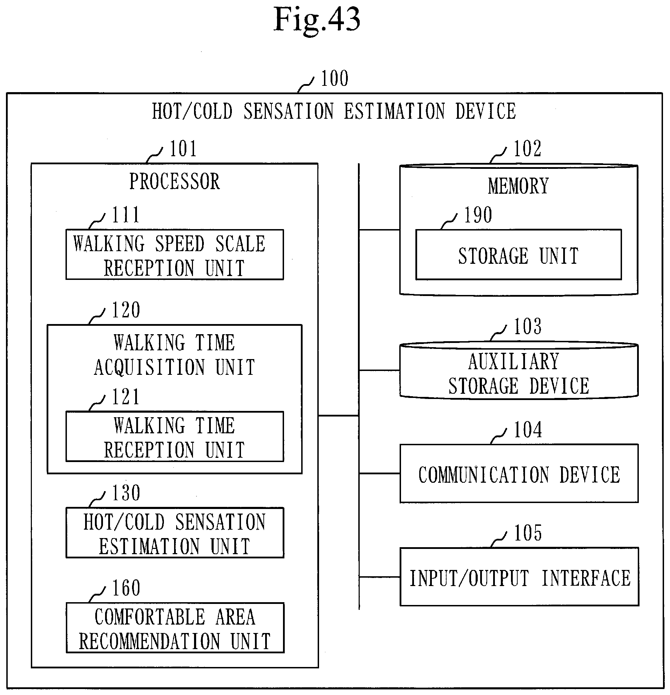

An embodiment for recommending a comfortable area in accordance with estimated hot/cold sensation will be described focusing mainly on differences from Embodiment 1, with referring to Fig. 43 to Fig. 46.

*** Description of Configuration ***

With referring to Fig. 43, a configuration of a hot/cold sensation estimation device 100 will be described.

The hot/cold sensation estimation device 100 is further provided with an element which is a comfortable area recommendation unit 160.

A hot/cold sensation estimation program further causes a computer to function as the comfortable area recommendation unit 160.

*** Description of Operation ***

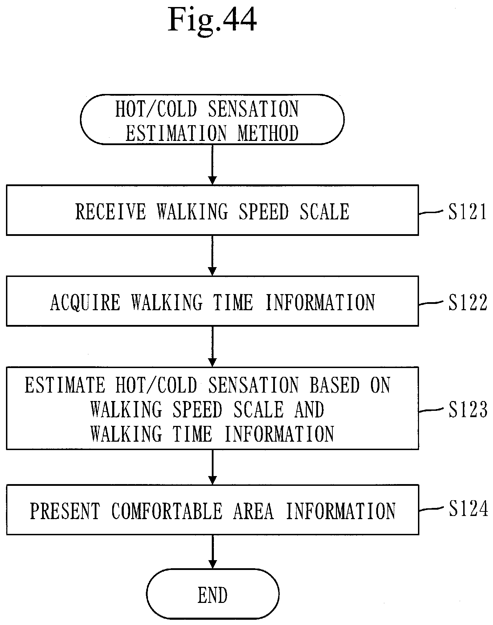

With referring to Fig. 44, a hot/cold sensation estimation method will be described.

In step S121, a walking speed scale reception unit 111 receives a walking speed scale inputted to the hot/cold sensation estimation device 100.

Step S121 is identical to step S011 of Embodiment 1.

In step S122, a walking environment acquisition unit 112 acquires walking time information.

Step S122 is identical to step S012 of Embodiment 1.

Steps S121 and S122 may be executed in a reverse order.

After steps S121 and S122, the processing proceeds to step S103.

In step S123, a hot/cold sensation estimation unit 130, using the received walking speed scale and the acquired walking time information as usage information, estimates hot/cold sensation felt by the user after walking based on the usage information.

Step S123 is identical to step S013 of Embodiment 1.

In step S124, the comfortable area recommendation unit 160 selects a comfortable area based on the estimated hot/cold sensation and a list of usage areas.

The comfortable area is one of a plurality of usage areas.

The list of usage areas shows, for each usage area, an area identifier and area environment information associated to each other.

The usage area is an area that can be used by the user after walking. For example, the plurality of usage areas are a plurality of rooms within a building that serves as a walking destination. For example, a room number is used as an area identifier.

The area environment information is data indicating an environment of the usage area. For instance, the area environment information indicates a setting temperature, setting humidity, and the like.

For example, if an estimated hot/cold sensation is higher than a certain level by a certain value or more, the comfortable area recommendation unit 160 selects, from the list of usage areas, an area identifier associated with area environment information indicating a setting temperature lower than the estimated hot/cool sensation by a certain value or more. The usage area identified by the selected area identifier serves as the comfortable area.

The comfortable area recommendation unit 160 may select the comfortable area by referring to information such as a travel distance from the entrance of the building to each area, the congestion level of each area, and area selection preference of the user. The congestion level indicates whether there are many people or not. The area selection preference indicates a particular area favored by the user, the length of the travel distance, the number of users, the level of humidity, and so on.

Then, the comfortable area recommendation unit 160 presents comfortable area information.

The comfortable area information is data indicating the selected comfortable area. For instance, the comfortable area information shows an area identifier of the comfortable area, and the like.

For instance, the comfortable area recommendation unit 160 displays the comfortable area information onto a display.

After step S124, the processing of a hot/cold sensation estimation method ends.

\*\*\* Effects of Embodiment 12 \*\*\*

Embodiment 12 recommends a comfortable area for the user based on the estimated hot/cold sensation and the area environment information. This enables suggesting a comfortable area based on the hot/cold sensation felt after walking.

\*\*\* Example of Embodiment 12 \*\*\*

Embodiment 12 may be applied to Embodiment 2.

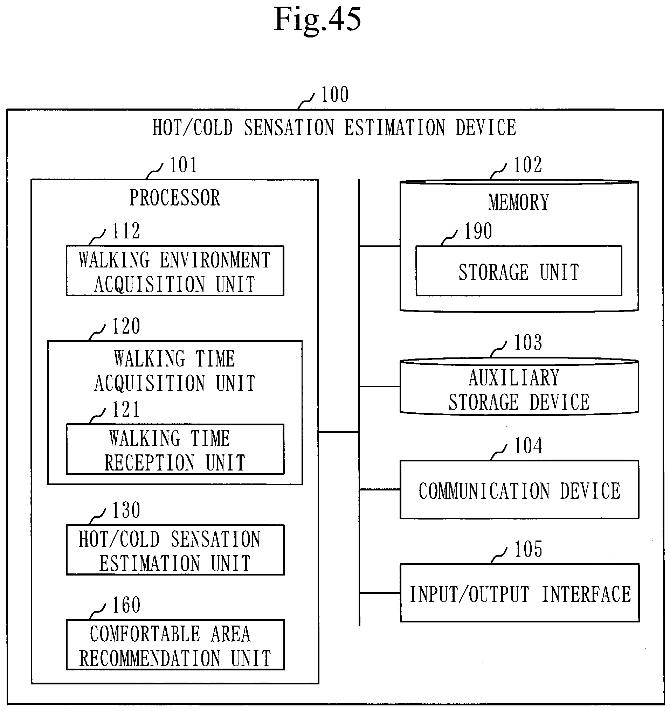

Fig. 45 shows a configuration of a hot/cold sensation estimation device 100 of a case where Embodiment 12 is applied to Embodiment 2.

The hot/cold sensation estimation device 100 is provided with a walking environment acquisition unit 112 instead of a walking speed scale reception unit 111.

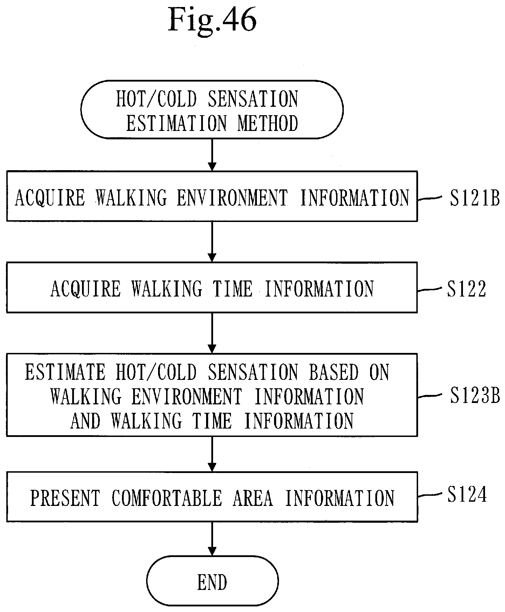

Fig. 46 shows a flowchart of a hot/cold sensation estimation method of the case where Embodiment 11 is applied to Embodiment 2.

In step S121B, the walking environment acquisition unit 112 acquires walking environment information. Step S101B is identical to step S021 in Embodiment 2.

Step S122 is as described in Embodiment 12.

In step S123B, a hot/cold sensation estimation unit 130, using the acquired walking environment information and acquired walking time information as usage information, estimates hot/cold sensation felt by the user after walking based on the usage information. Step S123B is identical to step S023 of Embodiment 2.

Step S124 is as described in Embodiment 12.

\*\*\* Supplementary Explanation of Embodiments \*\*\*

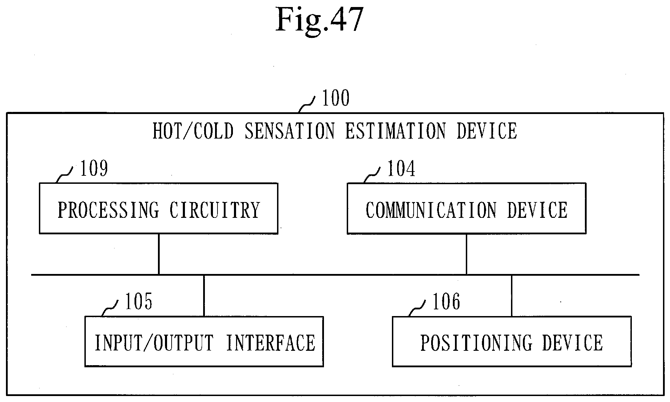

With referring to Fig. 47, a hardware configuration of the hot/cold sensation estimation device 100 will be described.

The hot/cold sensation estimation device 100 is provided with a processing circuitry 109.

The processing circuitry 109 is hardware that implements the walking speed scale reception unit 111, the walking time acquisition unit 120, and the hot/cold sensation estimation unit 130. Furthermore, the processing circuitry 109 implements elements such as the walking environment acquisition unit 112, the walking attribute reception unit 141, the walking date and time acquisition unit 142, the beacon information receiving unit 143, the positioning function control unit 144, the equipment control unit 151, the neighborhood equipment identification unit 152, and the comfortable area recommendation unit 160.

The processing circuitry 109 can be dedicated hardware, or can be the processor 101 that executes a program stored in the memory 102.

If the processing circuitry 109 is a dedicated hardware, the processing circuitry 109 is, for example, a single circuit, a composite circuit, a programmed processor, a parallel-programmed processor, an ASIC, an FPGA, or a combination of these.

Note that ASIC is short for Application Specific Integrated Circuit.

Note that FPGA is short for Field Programmable Gate Array.

The hot/cold sensation estimation device 100 may be provided with a plurality of processing circuits as an alternative to the processing circuitry 109.

In the processing circuitry 109, some functions may be implemented by dedicated hardware, while the remaining functions may be implemented by software or firmware.

In this manner, the functions of the hot/cold sensation estimation device 100 can be implemented by hardware, software, firmware, or a combination of these.

Each embodiment is merely an exemplification of a preferable embodiment and does not intend to limit the technical scope of the present disclosure. Each embodiment may be implemented partly, or may be implemented in combination with another embodiment. Two or more embodiments (or examples) may be implemented in combination. The procedures described using the flowcharts or the like may be suitably modified.

The word "unit" in the term of each element of the hot/cold sensation estimation device 100 may be replaced with "process", "stage", "circuit", or "circuitry".

## Reference Signs List

100: hot/cold sensation estimation device; 101: processor; 102: memory; 103: auxiliary storage device; 104: communication device; 105: input/output interface; 106: positioning device; 109: processing circuitry; 111: walking speed scale reception unit; 112: walking environment acquisition unit; 120: walking time acquisition unit; 121: walking time reception unit; 122: walking time calculation unit; 130: hot/cold sensation estimation unit; 141: walking attribute reception unit; 142: walking date and time acquisition unit; 143: beacon information receiving unit; 144: positioning function control unit; 151: equipment control unit; 152: neighborhood equipment identification unit; 160: comfortable area recommendation unit; 190: storage unit.

## Claims

1. A hot/cold sensation estimation device comprising:

   a walking speed scale reception unit to receive a walking speed scale inputted as a scale that represents a walking speed of a user;
   a walking time acquisition unit to acquire walking time information representing, as walking time, a duration of time for which the user was walking; and
   a hot/cold sensation estimation unit to estimate, using the received walking speed scale and the acquired walking time information as usage information, hot/cold sensation felt by the user after walking based on the usage information.

2. A hold/cold sensation estimation device comprising:

   a walking environment acquisition unit to acquire walking environment information representing an environment of a walking region where walking by a user takes place;
   a walking time acquisition unit to acquire walking time information representing, as walking time, a duration of time for which the user was walking; and
   a hot/cold sensation estimation unit to estimate, using the acquired walking environment information and the acquired walking time information as usage information, hot/cold sensation felt by the user after walking based on the usage information.

3. The hold/cold sensation estimation device according to claim 1 or 2, comprising:

   a walking attribute reception unit to receive walking attribute information inputted as information indicating attributes related to walking of the user,
   wherein the hot/cold sensation estimation unit includes the received walking attribute information into the usage information and estimates the hot/cold sensation based on the usage information.

4. The hot/cold sensation estimation device according to any one of claims 1 to 3, comprising:

   a walking date and time acquisition unit to acquire walking date and time information indicating a date and time of walking corresponding to a date and time when the user was walking,
   wherein the hot/cold sensation estimation unit includes the acquired walking date and time information into the usage information and estimates the hot/cold sensation based on the usage information.

5. The hot/cold sensation estimation device according to claim 1, comprising:

   a walking environment acquisition unit to acquire walking environment information representing an environment of a walking region where walking by the user takes place,
   wherein the hot/cold sensation estimation unit includes the acquired walking environment information into the usage information and estimates the hot/cold sensation based on the usage information.

6. The hot/cold sensation estimation device according to claim 2 or 5,

   the hot/cold sensation estimation device being a user terminal possessed by the user when the user walks,
   wherein the walking environment acquisition unit acquires environment information of a region corresponding to location information of the user terminal as the walking environment information.

7. The hot/cold sensation estimation device according to any one of claims 1 to 6, wherein the walking time reception unit receives the inputted walking time information using a user interface.

8. The hot/cold sensation estimation device according to any one of claims 1 to 6,

the hot/cold sensation estimation device being a user terminal possessed by the user when the user walks,

wherein the walking time acquisition unit obtains, as a walking start time point, a time point when the user terminal has entered a walking start zone, based on walking zone information indicating the walking start zone and a walking end zone, and location information of the user terminal at each time point,

obtains, as a walking end time point, a time point when the user terminal has entered the walking end zone, based on the walking zone information and the location information of the user terminal at each time point, and

calculates the walking time using the walking start time point and the walking end time point.

9. The hot/cold sensation estimation device according to any one of claims 1 to 6,

the hot/cold sensation estimation device being a user terminal possessed by the user when the user walks and comprising a beacon information receiving unit,

wherein the beacon information receiving unit receives beacon information emitted by a beacon provided to a walking end zone, and

wherein the walking time acquisition unit obtains, as a walking start time point, a time point when the user terminal has entered a walking start zone, based on walking zone information indicating the walking start zone, and location information of the user terminal at each time point,

obtains, as a walking end time point, a time point when the beacon information has been received, and

calculates the walking time using the walking start time point and the walking end time point.

10. The hot/cold sensation estimation device according to claim 9, comprising:
a positioning function control unit to turn off a positioning function of the user terminal when the user terminal has entered the walking start zone.

11. The hot/cold sensation estimation device according to any one of claims 1 to 10, comprising:

an equipment control unit to control designated equipment that is designated as one of pieces of heating/cooling equipment that affect human hot/cold sensation,

wherein the equipment control unit controls the designated equipment based on the estimated hot/cold sensation.

12. The hot/cold sensation estimation device according to claim 11,
wherein the equipment control unit adjusts setting of the designated equipment based on the estimated hot/cold sensation and favorite setting of the user for the designated equipment.

13. The hot/cold sensation estimation device according to any one of claims 1 to 10, comprising:

a neighborhood equipment identification unit to receive beacon information from at least one of a plurality of pieces of heating/cooling equipment which are located at positions different from each other and which affect human hot/cold sensation, and to identify, as neighborhood equipment, one of the plurality of pieces of heating/cooling equipment based on a reception result of the beacon information; and

an equipment control unit to control the neighborhood equipment based on the estimated hot/cold sensation.

14. The hot/cold sensation estimation device according to any one of claims 1 to 10, comprising:

a comfortable area recommendation unit to select, as a comfortable area, one of a plurality of usage areas that can be used by the user after walking, and to present comfortable area information indicating the selected comfortable area, wherein the comfortable area recommendation unit selects the comfortable area based on the estimated hot/cold sensation and area environment information indicating an environment of each of the usage areas.

15. A hot/cold sensation estimation method comprising:

receiving a walking speed scale inputted as a scale that represents a walking speed of a user; acquiring walking time information representing, as walking time, a duration of time for which the user was walking; and

estimating, using the received walking speed scale and the acquired walking time information as usage information, hot/cold sensation felt by the user after walking based on the usage information.

16. A hold/cold sensation estimation method comprising:

acquiring walking environment information representing an environment of a walking region where walking by a user takes place; acquiring walking time information representing, as walking time, a duration of time for which the user was walking; and
estimating, using the acquired walking environment information and the acquired walking time information as usage information, hot/cold sensation felt by the user after walking based on the usage information.

17. A hot/cold sensation estimation program which causes a computer to execute:

a walking speed scale reception process of receiving a walking speed scale inputted as a scale that represents a walking speed of a user;
a walking time acquisition process of acquiring walking time information representing, as walking time, a duration of time for which the user was walking; and
a hot/cold sensation estimation process of estimating, using the received walking speed scale and the acquired walking time information as usage information, hot/cold sensation felt by the user after walking based on the usage information.

18. A hold/cold sensation estimation program which causes a computer to execute:

a walking environment acquisition process of acquiring walking environment information representing an environment of a walking region where walking by a user takes place;
a walking time acquisition process of acquiring walking time information representing, as walking time, a duration of time for which the user was walking; and
a hot/cold sensation estimation process of estimating, using the acquired walking environment information and the acquired walking time information as usage information, hot/cold sensation felt by the user after walking based on the usage information.

# Fig.1

**100**
HOT/COLD SENSATION ESTIMATION DEVICE

**101**
PROCESSOR

**111**
WALKING SPEED SCALE RECEPTION UNIT

**120**
WALKING TIME ACQUISITION UNIT

**121**
WALKING TIME RECEPTION UNIT

**130**
HOT/COLD SENSATION ESTIMATION UNIT

**102**
MEMORY

**190**
STORAGE UNIT

**103**
AUXILIARY STORAGE DEVICE

**104**
COMMUNICATION DEVICE

**105**
INPUT/OUTPUT INTERFACE

# Fig.2

```
┌─────────────────────────┐
│    HOT/COLD SENSATION    │
│    ESTIMATION METHOD     │
└─────────────────────────┘
             │
             ▼
┌─────────────────────────────────────┐
│     RECEIVE WALKING SPEED SCALE      │──── S011
└─────────────────────────────────────┘
             │
             ▼
┌─────────────────────────────────────┐
│   ACQUIRE WALKING TIME INFORMATION   │──── S012
└─────────────────────────────────────┘
             │
             ▼
┌─────────────────────────────────────┐
│  ESTIMATE HOT/COLD SENSATION BASED   │
│    ON WALKING SPEED SCALE AND        │──── S013
│     WALKING TIME INFORMATION         │
└─────────────────────────────────────┘
             │
             ▼
         ┌─────────┐
         │   END   │
         └─────────┘
```

# Fig.3

LOCATION AT
TIME POINT t1

LOCATION AT
TIME POINT t2

100m

100m

DISTANCE
BETWEEN
2 POINTS

ERROR RANGE
OF LOCATION
INFORMATION

# Fig.4

100

HOT/COLD SENSATION ESTIMATION DEVICE

101
PROCESSOR

112
WALKING ENVIRONMENT
ACQUISITION UNIT

120
WALKING TIME
ACQUISITION UNIT
121
WALKING TIME
RECEPTION UNIT

130
HOT/COLD SENSATION
ESTIMATION UNIT

102
MEMORY
190
STORAGE UNIT

103
AUXILIARY
STORAGE DEVICE

104
COMMUNICATION DEVICE

105
INPUT/OUTPUT INTERFACE

# Fig.5

```
        ┌─────────────────────────────┐
        │      HOT/COLD SENSATION      │
        │      ESTIMATION METHOD       │
        └─────────────────────────────┘
                      │
                      ▼
   ┌────────────────────────────────────────────┐
   │  ACQUIRE WALKING ENVIRONMENT INFORMATION    │─── S021
   └────────────────────────────────────────────┘
                      │
                      ▼
   ┌────────────────────────────────────────────┐
   │     ACQUIRE WALKING TIME INFORMATION        │─── S022
   └────────────────────────────────────────────┘
                      │
                      ▼
   ┌────────────────────────────────────────────┐
   │   ESTIMATE HOT/COLD SENSATION BASED ON      │
   │     WALKING ENVIRONMENT INFORMATION         │─── S023
   │      AND WALKING TIME INFORMATION           │
   └────────────────────────────────────────────┘
                      │
                      ▼
              ┌───────────────┐
              │      END      │
              └───────────────┘
```

# Fig.6

HOT/COLD SENSATION ESTIMATION DEVICE
/ 100

PROCESSOR
/ 101

WALKING SPEED SCALE
RECEPTION UNIT
/ 111

WALKING TIME
ACQUISITION UNIT
/ 120

WALKING TIME
RECEPTION UNIT
/ 121

HOT/COLD SENSATION
ESTIMATION UNIT
/ 130

WALKING ATTRIBUTE
RECEPTION UNIT
/ 141

MEMORY
/ 102

STORAGE UNIT
/ 190

AUXILIARY
STORAGE DEVICE
/ 103

COMMUNICATION DEVICE
/ 104

INPUT/OUTPUT INTERFACE
/ 105

# Fig.7

```
┌─────────────────────────┐
│    HOT/COLD SENSATION    │
│    ESTIMATION METHOD     │
└─────────────────────────┘
             │
             ▼
┌─────────────────────────────────────────┐
│      RECEIVE WALKING SPEED SCALE         │──── S031
└─────────────────────────────────────────┘
             │
             ▼
┌─────────────────────────────────────────┐
│     ACQUIRE WALKING TIME INFORMATION     │──── S032
└─────────────────────────────────────────┘
             │
             ▼
┌─────────────────────────────────────────┐
│   RECEIVE WALKING ATTRIBUTE INFORMATION  │──── S033
└─────────────────────────────────────────┘
             │
             ▼
┌─────────────────────────────────────────┐
│ ESTIMATE HOT/COLD SENSATION BASED ON     │
│ WALKING SPEED SCALE, WALKING TIME        │──── S034
│ INFORMATION, AND WALKING ATTRIBUTE       │
│ INFORMATION                              │
└─────────────────────────────────────────┘
             │
             ▼
       ┌───────────┐
       │    END    │
       └───────────┘
```

# Fig.8

/100

## HOT/COLD SENSATION ESTIMATION DEVICE

/101

### PROCESSOR

/112
WALKING ENVIRONMENT
ACQUISITION UNIT

/120
WALKING TIME
ACQUISITION UNIT

/121
WALKING TIME
RECEPTION UNIT

/130
HOT/COLD SENSATION
ESTIMATION UNIT

/141
WALKING ATTRIBUTE
RECEPTION UNIT

/102
MEMORY

/190
STORAGE UNIT

/103
AUXILIARY
STORAGE DEVICE

/104
COMMUNICATION DEVICE

/105
INPUT/OUTPUT INTERFACE

# Fig.9

```
┌─────────────────────────┐
│    HOT/COLD SENSATION    │
│    ESTIMATION METHOD     │
└─────────────────────────┘
            │
            ▼
┌─────────────────────────────────────────────┐
│ AACQUIRE WALKING ENVIRONMENT INFORMATION │──── S031B
└─────────────────────────────────────────────┘
            │
            ▼
┌─────────────────────────────────────────────┐
│      ACQUIRE WALKING TIME INFORMATION        │──── S032
└─────────────────────────────────────────────┘
            │
            ▼
┌─────────────────────────────────────────────┐
│     RECEIVE WALKING ATTRIBUTE INFORMATION    │──── S033
└─────────────────────────────────────────────┘
            │
            ▼
┌─────────────────────────────────────────────┐
│     ESTIMATE HOT/COLD SENSATION BASED ON     │
│       WALKING ENVIRONMENT INFORMATION,       │──── S034B
│          WALKING TIME INFORMATION,           │
│      AND WALKING ATTRIBUTE INFORMATION       │
└─────────────────────────────────────────────┘
            │
            ▼
       ┌─────────┐
       │   END   │
       └─────────┘
```

# Fig.10

100

## HOT/COLD SENSATION ESTIMATION DEVICE

### 101
#### PROCESSOR

##### 111
WALKING SPEED SCALE
RECEPTION UNIT

##### 120
WALKING TIME
ACQUISITION UNIT

###### 121
WALKING TIME
RECEPTION UNIT

##### 130
HOT/COLD SENSATION
ESTIMATION UNIT

##### 142
WALKING DATE AND TIME
ACQUISITION UNIT

### 102
#### MEMORY

##### 190
STORAGE UNIT

### 103
AUXILIARY
STORAGE DEVICE

### 104
COMMUNICATION DEVICE

### 105
INPUT/OUTPUT INTERFACE

# Fig.11

```
┌─────────────────────────────┐
│      HOT/COLD SENSATION      │
│      ESTIMATION METHOD       │
└─────────────────────────────┘
              │
              ▼
┌─────────────────────────────┐
│  RECEIVE WALKING SPEED SCALE │──── S041
└─────────────────────────────┘
              │
              ▼
┌─────────────────────────────┐
│ ACQUIRE WALKING TIME INFORMATION │──── S042
└─────────────────────────────┘
              │
              ▼
┌─────────────────────────────┐
│    ACQUIRE WALKING DATE AND   │
│       TIME INFORMATION        │──── S043
└─────────────────────────────┘
              │
              ▼
┌─────────────────────────────┐
│  ESTIMATE HOT/COLD SENSATION BASED │
│   ON WALKING SPEED SCALE, WALKING  │──── S044
│  TIME INFORMATION, AND WALKING DATE│
│        AND TIME INFORMATION        │
└─────────────────────────────┘
              │
              ▼
         ┌─────────┐
         │   END   │
         └─────────┘
```

# Fig.12

```
                                      ⟋100
┌─────────────────────────────────────────────────────────────────────┐
│              HOT/COLD SENSATION ESTIMATION DEVICE                     │
│        ⟋101                              ⟋102                          │
│  ┌──────────────────────────┐     ┌──────────────────────────────┐   │
│  │        PROCESSOR          │     │          MEMORY              │   │
│  │     ⟋112                  │     │      ⟋190                    │   │
│  │  ┌────────────────────┐   │     │  ┌────────────────────────┐  │   │
│  │  │WALKING ENVIRONMENT │   │     │  │     STORAGE UNIT        │  │   │
│  │  │ACQUISITION UNIT    │   │     │  └────────────────────────┘  │   │
│  │  └────────────────────┘   │     └──────────────────────────────┘   │
│  │     ⟋120                  │                                        │
│  │  ┌────────────────────┐   │           ⟋103                         │
│  │  │   WALKING TIME     │   │     ┌──────────────────────────────┐   │
│  │  │ ACQUISITION UNIT   │   │     │       AUXILIARY              │   │
│  │  │    ⟋121            │   │     │   STORAGE DEVICE             │   │
│  │  │ ┌───────────────┐  │   │     └──────────────────────────────┘   │
│  │  │ │ WALKING TIME  │  │   │                                        │
│  │  │ │RECEPTION UNIT │  │   │           ⟋104                         │
│  │  │ └───────────────┘  │   │     ┌──────────────────────────────┐   │
│  │  └────────────────────┘   │     │   COMMUNICATION DEVICE       │   │
│  │     ⟋130                  │     └──────────────────────────────┘   │
│  │  ┌────────────────────┐   │                                        │
│  │  │ HOT/COLD SENSATION │   │           ⟋105                         │
│  │  │  ESTIMATION UNIT   │   │     ┌──────────────────────────────┐   │
│  │  └────────────────────┘   │     │  INPUT/OUTPUT INTERFACE      │   │
│  │     ⟋142                  │     └──────────────────────────────┘   │
│  │  ┌────────────────────┐   │                                        │
│  │  │WALKING DATE AND TIME│  │                                        │
│  │  │ ACQUISITION UNIT   │   │                                        │
│  │  └────────────────────┘   │                                        │
│  └──────────────────────────┘                                        │
└─────────────────────────────────────────────────────────────────────┘
```

# Fig.13

```
┌─────────────────────────┐
│   HOT/COLD SENSATION    │
│   ESTIMATION METHOD     │
└─────────────────────────┘
             │
             ▼
┌─────────────────────────────────────────┐
│ ACQUIRE WALKING ENVIRONMENT INFORMATION  │── S041B
└─────────────────────────────────────────┘
             │
             ▼
┌─────────────────────────────────────────┐
│   ACQUIRE WALKING TIME INFORMATION       │── S042
└─────────────────────────────────────────┘
             │
             ▼
┌─────────────────────────────────────────┐
│       ACQUIRE WALKING DATE AND           │── S043
│         TIME INFORMATION                 │
└─────────────────────────────────────────┘
             │
             ▼
┌─────────────────────────────────────────┐
│   ESTIMATE HOT/COLD SENSATION BASED      │
│ ON WALKING ENVIRONMENT INFORMATION,      │── S044B
│    WALKING TIME INFORMATION, AND         │
│  WALKING DATE AND TIME INFORMATION       │
└─────────────────────────────────────────┘
             │
             ▼
         ┌─────────┐
         │   END   │
         └─────────┘
```

# Fig.14

100

## HOT/COLD SENSATION ESTIMATION DEVICE

### 101
### PROCESSOR

#### 111
WALKING SPEED SCALE
RECEPTION UNIT

#### 120
WALKING TIME
ACQUISITION UNIT

##### 121
WALKING TIME
RECEPTION UNIT

#### 130
HOT/COLD SENSATION
ESTIMATION UNIT

#### 112
WALKING ENVIRONMENT
ACQUISITION UNIT

### 102
### MEMORY

#### 190
STORAGE UNIT

### 103
AUXILIARY
STORAGE DEVICE

### 104
COMMUNICATION DEVICE

### 105
INPUT/OUTPUT INTERFACE

# Fig.15

```
┌─────────────────────────┐
│   HOT/COLD SENSATION     │
│   ESTIMATION METHOD      │
└─────────────────────────┘
             │
             ▼
┌─────────────────────────────────────┐
│   RECEIVE WALKING SPEED SCALE        │──── S051
└─────────────────────────────────────┘
             │
             ▼
┌─────────────────────────────────────┐
│   ACQUIRE WALKING TIME INFORMATION   │──── S052
└─────────────────────────────────────┘
             │
             ▼
┌──────────────────────────────────────────┐
│   ACQUIRE WALKING ENVIRONMENT INFORMATION │──── S053
└──────────────────────────────────────────┘
             │
             ▼
┌─────────────────────────────────────┐
│   ESTIMATE HOT/COLD SENSATION BASED  │
│       ON WALKING SPEED SCALE,        │──── S054
│    WALKING TIME INFORMATION, AND     │
│   WALKING ENVIRONMENT INFORMATION    │
└─────────────────────────────────────┘
             │
             ▼
        ┌─────────┐
        │   END   │
        └─────────┘
```

# Fig.16

# Fig.17

```
┌─────────────────────────┐
│   HOT/COLD SENSATION     │
│   ESTIMATION METHOD      │
└─────────────────────────┘
            │
            ▼
┌─────────────────────────────────┐
│   RECEIVE WALKING SPEED SCALE   │──── S061
└─────────────────────────────────┘
            │
            ▼
┌─────────────────────────────────┐
│  ACQUIRE WALKING TIME INFORMATION │──── S062
└─────────────────────────────────┘
            │
            ▼
┌──────────────────────────────────────┐
│ ACQUIRE WALKING ENVIRONMENT INFORMATION │──── S063
└──────────────────────────────────────┘
            │
            ▼
┌──────────────────────────────────────┐
│   ESTIMATE HOT/COLD SENSATION BASED   │
│       ON WALKING SPEED SCALE,         │──── S064
│       WALKING TIME INFORMATION,       │
│   AND WALKING ENVIRONMENT INFORMATION │
└──────────────────────────────────────┘
            │
            ▼
      ┌───────────┐
      │    END    │
      └───────────┘
```

# Fig.18

100

## HOT/COLD SENSATION ESTIMATION DEVICE

### 101
#### PROCESSOR

##### 112
###### WALKING ENVIRONMENT ACQUISITION UNIT

##### 120
###### WALKING TIME ACQUISITION UNIT

###### 121
####### WALKING TIME RECEPTION UNIT

##### 130
###### HOT/COLD SENSATION ESTIMATION UNIT

### 102
#### MEMORY

##### 190
###### STORAGE UNIT

### 103
#### AUXILIARY STORAGE DEVICE

### 104
#### COMMUNICATION DEVICE

### 105
#### INPUT/OUTPUT INTERFACE

### 106
#### POSITIONING DEVICE

# Fig.19

```
          ┌─────────────────────────┐
          │     HOT/COLD SENSATION   │
          │     ESTIMATION METHOD    │
          └─────────────────────────┘
                       │
                       ▼
    ┌──────────────────────────────────────────┐
    │ ACQUIRE WALKING ENVIRONMENT INFORMATION   │── S063
    └──────────────────────────────────────────┘
                       │
                       ▼
    ┌──────────────────────────────────────────┐
    │    ACQUIRE WALKING TIME INFORMATION       │── S062
    └──────────────────────────────────────────┘
                       │
                       ▼
    ┌──────────────────────────────────────────┐
    │   ESTIMATE HOT/COLD SENSATION BASED ON    │
    │   WALKING ENVIRONMENT INFORMATION         │── S064B
    │   AND WALKING TIME INFORMATION            │
    └──────────────────────────────────────────┘
                       │
                       ▼
                 ┌───────────┐
                 │    END     │
                 └───────────┘
```

# Fig.20

```
                              ⟋100
┌──────────────────────────────────────────────────────────────────────┐
│              HOT/COLD SENSATION ESTIMATION DEVICE                      │
│      ⟋101                               ⟋102                           │
│  ┌─────────────────────────┐       ┌──────────────────────────────┐  │
│  │       PROCESSOR          │       │           MEMORY             │  │
│  │    ⟋111                  │       │       ⟋190                   │  │
│  │ ┌─────────────────────┐  │       │  ┌────────────────────────┐  │  │
│  │ │WALKING SPEED SCALE  │  │       │  │     STORAGE UNIT       │  │  │
│  │ │ RECEPTION UNIT      │  │       │  └────────────────────────┘  │  │
│  │ └─────────────────────┘  │       └──────────────────────────────┘  │
│  │    ⟋120                  │                                         │
│  │ ┌─────────────────────┐  │           ⟋103                          │
│  │ │    WALKING TIME     │  │       ┌──────────────────────────────┐  │
│  │ │  ACQUISITION UNIT   │  │       │        AUXILIARY             │  │
│  │ │    ⟋122             │  │       │     STORAGE DEVICE           │  │
│  │ │ ┌─────────────────┐ │  │       └──────────────────────────────┘  │
│  │ │ │ WALKING TIME    │ │  │                                         │
│  │ │ │CALCULATION UNIT │ │  │           ⟋104                          │
│  │ │ └─────────────────┘ │  │       ┌──────────────────────────────┐  │
│  │ └─────────────────────┘  │       │   COMMUNICATION DEVICE       │  │
│  │    ⟋130                  │       └──────────────────────────────┘  │
│  │ ┌─────────────────────┐  │                                         │
│  │ │ HOT/COLD SENSATION  │  │           ⟋105                          │
│  │ │  ESTIMATION UNIT    │  │       ┌──────────────────────────────┐  │
│  │ └─────────────────────┘  │       │   INPUT/OUTPUT INTERFACE     │  │
│  └─────────────────────────┘       └──────────────────────────────┘  │
│                                                                        │
│                                         ⟋106                          │
│                                     ┌──────────────────────────────┐  │
│                                     │   POSITIONING DEVICE         │  │
│                                     └──────────────────────────────┘  │
└──────────────────────────────────────────────────────────────────────┘
```

# Fig.21

```
   ┌─────────────────────────────┐
   │   HOT/COLD SENSATION        │
   │   ESTIMATION METHOD         │
   └─────────────────────────────┘
                │
                ▼
   ┌─────────────────────────────┐
   │   RECEIVE WALKING SPEED SCALE│──── S071
   └─────────────────────────────┘
                │
                ▼
   ┌─────────────────────────────┐
   │  ACQUIRE WALKING TIME INFORMATION│──── S072
   └─────────────────────────────┘
                │
                ▼
   ┌─────────────────────────────┐
   │ ESTIMATE HOT/COLD SENSATION BASED ON│──── S073
   │    WALKING SPEED SCALE AND  │
   │    WALKING TIME INFORMATION │
   └─────────────────────────────┘
                │
                ▼
           ┌─────────┐
           │   END   │
           └─────────┘
```

# Fig.22

WALKING END
ZONE

WALKING
START ZONE

WALKING END
POINT

WALKING
START POINT

# Fig.23

WALKING END
ZONE

WALKING END
POINT

WALKING
START ZONE

WALKING
START POINT

# Fig.24

HOT/COLD SENSATION ESTIMATION DEVICE — 100

PROCESSOR — 101
- WALKING ENVIRONMENT ACQUISITION UNIT — 112
- WALKING TIME ACQUISITION UNIT — 120
  - WALKING TIME CALCULATION UNIT — 122
- HOT/COLD SENSATION ESTIMATION UNIT — 130

MEMORY — 102
- STORAGE UNIT — 190

AUXILIARY STORAGE DEVICE — 103

COMMUNICATION DEVICE — 104

INPUT/OUTPUT INTERFACE — 105

POSITIONING DEVICE — 106

# Fig.25

```
         ┌─────────────────────────┐
         │   HOT/COLD SENSATION     │
         │   ESTIMATION METHOD      │
         └─────────────────────────┘
                    │
                    ▼
   ┌────────────────────────────────────────┐
   │ ACQUIRE WALKING ENVIRONMENT INFORMATION │──── S071B
   └────────────────────────────────────────┘
                    │
                    ▼
   ┌────────────────────────────────────────┐
   │   ACQUIRE WALKING TIME INFORMATION      │──── S072
   └────────────────────────────────────────┘
                    │
                    ▼
   ┌────────────────────────────────────────┐
   │  ESTIMATE HOT/COLD SENSATION BASED ON   │
   │   WALKING ENVIRONMENT INFORMATION       │──── S073B
   │     AND WALKING TIME INFORMATION        │
   └────────────────────────────────────────┘
                    │
                    ▼
              ┌──────────┐
              │   END    │
              └──────────┘
```

# Fig.26

HOT/COLD SENSATION ESTIMATION DEVICE — 100

PROCESSOR — 101

- WALKING SPEED SCALE RECEPTION UNIT — 111
- WALKING TIME ACQUISITION UNIT — 120
  - WALKING TIME CALCULATION UNIT — 122
- HOT/COLD SENSATION ESTIMATION UNIT — 130
- BEACON INFORMATION RECEIVING UNIT — 143

MEMORY — 102
- STORAGE UNIT — 190

AUXILIARY STORAGE DEVICE — 103

COMMUNICATION DEVICE — 104

INPUT/OUTPUT INTERFACE — 105

POSITIONING DEVICE — 106

# Fig.27

```
┌─────────────────────────┐
│   HOT/COLD SENSATION     │
│   ESTIMATION METHOD      │
└─────────────────────────┘
            │
            ▼
┌─────────────────────────────────┐
│  RECEIVE WALKING SPEED SCALE    │──── S081
└─────────────────────────────────┘
            │
            ▼
┌─────────────────────────────────┐
│  ACQUIRE WALKING TIME INFORMATION │──── S082
└─────────────────────────────────┘
            │
            ▼
┌─────────────────────────────────┐
│ ESTIMATE HOT/COLD SENSATION BASED ON │
│      WALKING SPEED SCALE         │──── S083
│  AND WALKING TIME INFORMATION    │
└─────────────────────────────────┘
            │
            ▼
      ┌───────────┐
      │    END    │
      └───────────┘
```

# Fig.28

WALKING
START ZONE

WALKING
START POINT

WALKING END
POINT

# Fig.29

100

## HOT/COLD SENSATION ESTIMATION DEVICE

### 101

#### PROCESSOR

##### 112
WALKING ENVIRONMENT
ACQUISITION UNIT

##### 120
WALKING TIME
ACQUISITION UNIT

##### 122
WALKING TIME
CALCULATION UNIT

##### 130
HOT/COLD SENSATION
ESTIMATION UNIT

##### 143
BEACON INFORMATION
RECEIVING UNIT

### 102
#### MEMORY

##### 190
STORAGE UNIT

### 103
AUXILIARY
STORAGE DEVICE

### 104
COMMUNICATION DEVICE

### 105
INPUT/OUTPUT INTERFACE

### 106
POSITIONING DEVICE

# Fig.30

```
        ┌─────────────────────────┐
        │    HOT/COLD SENSATION    │
        │    ESTIMATION METHOD     │
        └─────────────────────────┘
                     │
                     ▼
┌─────────────────────────────────────────────┐
│ ACQUIRE WALKING ENVIRONMENT INFORMATION      │──── S081B
└─────────────────────────────────────────────┘
                     │
                     ▼
┌─────────────────────────────────────────────┐
│     ACQUIRE WALKING TIME INFORMATION         │──── S082
└─────────────────────────────────────────────┘
                     │
                     ▼
┌─────────────────────────────────────────────┐
│  ESTIMATE HOT/COLD SENSATION BASED ON        │
│    WALKING ENVIRONMENT INFORMATION           │──── S083B
│      AND WALKING TIME INFORMATION            │
└─────────────────────────────────────────────┘
                     │
                     ▼
             ┌──────────────┐
             │     END      │
             └──────────────┘
```

# Fig.31

/ 100

**HOT/COLD SENSATION ESTIMATION DEVICE**

/ 101

**PROCESSOR**

/ 111

| WALKING SPEED SCALE RECEPTION UNIT |

/ 120

| WALKING TIME ACQUISITION UNIT |

/ 122

| WALKING TIME CALCULATION UNIT |

/ 130

| HOT/COLD SENSATION ESTIMATION UNIT |

/ 143

| BEACON INFORMATION RECEIVING UNIT |

/ 144

| POSITIONING FUNCTION CONTROL UNIT |

/ 102

**MEMORY**

/ 190

| STORAGE UNIT |

/ 103

| AUXILIARY STORAGE DEVICE |

/ 104

| COMMUNICATION DEVICE |

/ 105

| INPUT/OUTPUT INTERFACE |

/ 106

| POSITIONING DEVICE |

# Fig.32

```
    ┌─────────────────────────────┐
    │   POSITIONING FUNCTION      │
    │       CONTROL (A)           │
    └─────────────────────────────┘
                   │
                   ▼
┌───────────────────────────────────────────────┐
│   DETECT ENTRY OF USER TERMINAL INTO WALKING   │ ── S091A
│ START ZONE, AND TURN OFF POSITIONING FUNCTION  │
│              OF USER TERMINAL                   │
└───────────────────────────────────────────────┘
                   │
                   ▼
┌───────────────────────────────────────────────┐
│          DETECT ENTRY OF USER TERMINAL          │ ── S092A
│            INTO WALKING END ZONE                │
└───────────────────────────────────────────────┘
                   │
                   ▼
┌───────────────────────────────────────────────┐
│   DETECT EXIT OF USER TERMINAL FROM WALKING    │ ── S093A
│  END ZONE, AND TURN ON POSITIONING FUNCTION    │
│              OF USER TERMINAL                   │
└───────────────────────────────────────────────┘
                   │
                   ▼
            ┌──────────────┐
            │     END      │
            └──────────────┘
```

# Fig.33

```
       ┌─────────────────────────┐
       │  POSITIONING FUNCTION   │
       │      CONTROL (B)         │
       └─────────────────────────┘
                   │
                   ▼
┌────────────────────────────────────────────────────┐
│ DETECT ENTRY OF USER TERMINAL INTO WALKING START ZONE,│ ── S091B
│  AND TURN OFF POSITIONING FUNCTION OF USER TERMINAL   │
└────────────────────────────────────────────────────┘
                   │
                   ▼
┌────────────────────────────────────────────────────┐
│            DETECT ENTRY OF USER TERMINAL             │ ── S092B
│              INTO WALKING END ZONE                   │
└────────────────────────────────────────────────────┘
                   │
                   ▼
┌────────────────────────────────────────────────────┐
│ DETECT EXIT OF USER TERMINAL FROM WALKING END ZONE,  │
│  AND TURN ON POSITIONING FUNCTION OF USER TERMINAL   │ ── S093B
│              IN RESTRICTION SETTING                  │
└────────────────────────────────────────────────────┘
                   │
                   ▼
┌────────────────────────────────────────────────────┐
│     DETECT APPROACH OF USER TERMINAL TO WALKING      │
│   START POINT, AND RESTORE POSITIONING FUNCTION      │ ── S094B
│    SETTING OF USER TERMINAL TO DEFAULT SETTING       │
└────────────────────────────────────────────────────┘
                   │
                   ▼
            ┌─────────────┐
            │     END     │
            └─────────────┘
```

# Fig.34

┌─────────────────────────────────────────────────────────────┐
│ ╱100                                                         │
│ HOT/COLD SENSATION ESTIMATION DEVICE                         │
│                                                              │
│ ┌──────────────────────────────┐    ┌──────────────────────┐│
│ │ ╱101                         │    │ ╱102                 ││
│ │ PROCESSOR                    │    │ MEMORY               ││
│ │                              │    │ ╱190                 ││
│ │ ┌──────────────────────────┐ │    │ ┌──────────────────┐ ││
│ │ │ ╱112                     │ │    │ │ STORAGE UNIT     │ ││
│ │ │ WALKING ENVIRONMENT      │ │    │ └──────────────────┘ ││
│ │ │ ACQUISITION UNIT         │ │    └──────────────────────┘│
│ │ └──────────────────────────┘ │                            │
│ │ ┌──────────────────────────┐ │    ┌──────────────────────┐│
│ │ │ ╱120                     │ │    │ ╱103                 ││
│ │ │ WALKING TIME             │ │    │ AUXILIARY            ││
│ │ │ ACQUISITION UNIT         │ │    │ STORAGE DEVICE       ││
│ │ │ ╱122                     │ │    └──────────────────────┘│
│ │ │ ┌──────────────────────┐ │ │                            │
│ │ │ │ WALKING TIME         │ │ │    ┌──────────────────────┐│
│ │ │ │ CALCULATION UNIT     │ │ │    │ ╱104                 ││
│ │ │ └──────────────────────┘ │ │    │ COMMUNICATION DEVICE ││
│ │ └──────────────────────────┘ │    └──────────────────────┘│
│ │ ┌──────────────────────────┐ │                            │
│ │ │ ╱130                     │ │    ┌──────────────────────┐│
│ │ │ HOT/COLD SENSATION       │ │    │ ╱105                 ││
│ │ │ ESTIMATION UNIT          │ │    │ INPUT/OUTPUT INTERFACE││
│ │ └──────────────────────────┘ │    └──────────────────────┘│
│ │ ┌──────────────────────────┐ │                            │
│ │ │ ╱143                     │ │    ┌──────────────────────┐│
│ │ │ BEACON INFORMATION       │ │    │ ╱106                 ││
│ │ │ RECEIVING UNIT           │ │    │ POSITIONING DEVICE   ││
│ │ └──────────────────────────┘ │    └──────────────────────┘│
│ │ ┌──────────────────────────┐ │                            │
│ │ │ ╱144                     │ │                            │
│ │ │ POSITIONING FUNCTION     │ │                            │
│ │ │ CONTROL UNIT             │ │                            │
│ │ └──────────────────────────┘ │                            │
│ └──────────────────────────────┘                            │
└─────────────────────────────────────────────────────────────┘

# Fig.35

100 HOT/COLD SENSATION ESTIMATION DEVICE

101 PROCESSOR

111 WALKING SPEED SCALE RECEPTION UNIT

120 WALKING TIME ACQUISITION UNIT

121 WALKING TIME RECEPTION UNIT

130 HOT/COLD SENSATION ESTIMATION UNIT

151 EQUIPMENT CONTROL UNIT

102 MEMORY

190 STORAGE UNIT

103 AUXILIARY STORAGE DEVICE

104 COMMUNICATION DEVICE

105 INPUT/OUTPUT INTERFACE

# Fig.36

```
        ┌─────────────────────────┐
        │   HOT/COLD SENSATION    │
        │    ESTIMATION METHOD    │
        └─────────────────────────┘
                     │
                     ▼
    ┌─────────────────────────────────┐
    │  RECEIVE WALKING SPEED SCALE    │─── S101
    └─────────────────────────────────┘
                     │
                     ▼
    ┌─────────────────────────────────┐
    │  ACQUIRE WALKING TIME INFORMATION │─── S102
    └─────────────────────────────────┘
                     │
                     ▼
    ┌─────────────────────────────────┐
    │ ESTIMATE HOT/COLD SENSATION BASED ON │
    │     WALKING SPEED SCALE AND     │─── S103
    │      WALKING TIME INFORMATION   │
    └─────────────────────────────────┘
                     │
                     ▼
    ┌─────────────────────────────────┐
    │   CONTROL DESIGNATED EQUIPMENT  │
    │   BASED ON HOT/HOLD SENSATION   │─── S104
    └─────────────────────────────────┘
                     │
                     ▼
            ┌─────────────┐
            │     END     │
            └─────────────┘
```

# Fig.37

# Fig.38

```
┌─────────────────────────┐
│   HOT/COLD SENSATION     │
│   ESTIMATION METHOD      │
└─────────────────────────┘
            │
            ▼
┌──────────────────────────────────────────────┐
│ ACQUIRE WALKING ENVIRONMENT INFORMATION       │──── S101B
└──────────────────────────────────────────────┘
            │
            ▼
┌──────────────────────────────────────────────┐
│ ACQUIRE WALKING TIME INFORMATION              │──── S102
└──────────────────────────────────────────────┘
            │
            ▼
┌──────────────────────────────────────────────┐
│ ESTIMATE HOT/COLD SENSATION BASED ON          │
│ WALKING ENVIRONMENT INFORMATION               │──── S103B
│ AND WALKING TIME INFORMATION                  │
└──────────────────────────────────────────────┘
            │
            ▼
┌──────────────────────────────────────────────┐
│ CONTROL DESIGNATED EQUIPMENT                  │──── S104
│ BASED ON HOT/COLD SENSATION                   │
└──────────────────────────────────────────────┘
            │
            ▼
        ┌─────────┐
        │   END   │
        └─────────┘
```

# Fig.39

/100

**HOT/COLD SENSATION ESTIMATION DEVICE**

/101

**PROCESSOR**

/111

WALKING SPEED SCALE
RECEPTION UNIT

/120

WALKING TIME
ACQUISITION UNIT

/121

WALKING TIME
RECEPTION UNIT

/130

HOT/COLD SENSATION
ESTIMATION UNIT

/151

EQUIPMENT
CONTROL UNIT

/152

NEIGHBORHOOD EQUIPMENT
IDENTIFICATION UNIT

/102

**MEMORY**

/190

STORAGE UNIT

/103

AUXILIARY
STORAGE DEVICE

/104

COMMUNICATION DEVICE

/105

INPUT/OUTPUT INTERFACE

# Fig.40

```
      ┌─────────────────────────┐
      │     HOT/COLD SENSATION   │
      │    ESTIMATION METHOD     │
      └─────────────────────────┘
                   │
                   ▼
      ┌─────────────────────────┐
      │ RECEIVE WALKING SPEED SCALE │──── S111
      └─────────────────────────┘
                   │
                   ▼
      ┌─────────────────────────┐
      │ ACQUIRE WALKING TIME INFORMATION │──── S112
      └─────────────────────────┘
                   │
                   ▼
      ┌─────────────────────────┐
      │ ESTIMATE HOT/COLD SENSATION BASED ON │
      │     WALKING SPEED SCALE AND          │──── S113
      │     WALKING TIME INFORMATION         │
      └─────────────────────────┘
                   │
                   ▼
      ┌─────────────────────────┐
      │ IDENTIFY NEIGHBORHOOD EQUIPMENT │──── S114
      └─────────────────────────┘
                   │
                   ▼
      ┌─────────────────────────┐
      │ CONTROL NEIGHBORHOOD EQUIPMENT │
      │  BASED ON HOT/COLD SENSATION   │──── S115
      └─────────────────────────┘
                   │
                   ▼
             ┌──────────┐
             │   END    │
             └──────────┘
```

# Fig.41

/ 100

## HOT/COLD SENSATION ESTIMATION DEVICE

/ 101

### PROCESSOR

/ 112

#### WALKING ENVIRONMENT ACQUISITION UNIT

/ 120

#### WALKING TIME ACQUISITION UNIT

/ 121

##### WALKING TIME RECEPTION UNIT

/ 130

#### HOT/COLD SENSATION ESTIMATION UNIT

/ 151

#### EQUIPMENT CONTROL UNIT

/ 152

#### NEIGHBORHOOD EQUIPMENT IDENTIFICATION UNIT

/ 102

### MEMORY

/ 190

#### STORAGE UNIT

/ 103

### AUXILIARY STORAGE DEVICE

/ 104

### COMMUNICATION DEVICE

/ 105

### INPUT/OUTPUT INTERFACE

# Fig.42

```
        ┌─────────────────────────┐
        │    HOT/COLD SENSATION    │
        │    ESTIMATION METHOD     │
        └─────────────────────────┘
                    │
                    ▼
   ┌─────────────────────────────────────────┐
   │ ACQUIRE WALKING ENVIRONMENT INFORMATION  │──── S111B
   └─────────────────────────────────────────┘
                    │
                    ▼
   ┌─────────────────────────────────────────┐
   │    ACQUIRE WALKING TIME INFORMATION      │──── S112
   └─────────────────────────────────────────┘
                    │
                    ▼
   ┌─────────────────────────────────────────┐
   │   ESTIMATE HOT/COLD SENSATION BASED ON   │
   │    WALKING ENVIRONMENT INFORMATION       │──── S113B
   │      AND WALKING TIME INFORMATION        │
   └─────────────────────────────────────────┘
                    │
                    ▼
   ┌─────────────────────────────────────────┐
   │     IDENTIFY NEIGHBORHOOD EQUIPMENT      │──── S114
   └─────────────────────────────────────────┘
                    │
                    ▼
   ┌─────────────────────────────────────────┐
   │     CONTROL NEIGHBORHOOD EQUIPMENT       │──── S115
   │     BASED ON HOT/COLD SENSATION          │
   └─────────────────────────────────────────┘
                    │
                    ▼
              ┌───────────┐
              │    END    │
              └───────────┘
```

# Fig.43

100
HOT/COLD SENSATION ESTIMATION DEVICE

101
PROCESSOR

111
WALKING SPEED SCALE RECEPTION UNIT

120
WALKING TIME ACQUISITION UNIT

121
WALKING TIME RECEPTION UNIT

130
HOT/COLD SENSATION ESTIMATION UNIT

160
COMFORTABLE AREA RECOMMENDATION UNIT

102
MEMORY

190
STORAGE UNIT

103
AUXILIARY STORAGE DEVICE

104
COMMUNICATION DEVICE

105
INPUT/OUTPUT INTERFACE

# Fig.44

```
┌─────────────────────────────┐
│      HOT/COLD SENSATION      │
│      ESTIMATION METHOD       │
└─────────────────────────────┘
               │
               ▼
┌─────────────────────────────┐
│  RECEIVE WALKING SPEED SCALE │──── S121
└─────────────────────────────┘
               │
               ▼
┌─────────────────────────────┐
│ ACQUIRE WALKING TIME INFORMATION │──── S122
└─────────────────────────────┘
               │
               ▼
┌─────────────────────────────────┐
│  ESTIMATE HOT/COLD SENSATION BASED ON │
│      WALKING SPEED SCALE AND          │──── S123
│      WALKING TIME INFORMATION         │
└─────────────────────────────────┘
               │
               ▼
┌─────────────────────────────────┐
│ PRESENT COMFORTABLE AREA INFORMATION │──── S124
└─────────────────────────────────┘
               │
               ▼
         ┌───────────┐
         │    END    │
         └───────────┘
```

# Fig.45

/ 100

## HOT/COLD SENSATION ESTIMATION DEVICE

/ 101

### PROCESSOR

/ 112

WALKING ENVIRONMENT
ACQUISITION UNIT

/ 120

WALKING TIME
ACQUISITION UNIT

/ 121

WALKING TIME
RECEPTION UNIT

/ 130

HOT/COLD SENSATION
ESTIMATION UNIT

/ 160

COMFORTABLE AREA
RECOMMENDATION UNIT

/ 102

### MEMORY

/ 190

STORAGE UNIT

/ 103

AUXILIARY
STORAGE DEVICE

/ 104

COMMUNICATION DEVICE

/ 105

INPUT/OUTPUT INTERFACE

# Fig.46

```
┌─────────────────────────┐
│    HOT/COLD SENSATION    │
│    ESTIMATION METHOD     │
└─────────────────────────┘
            │
            ▼
┌──────────────────────────────────────────────┐
│ ACQUIRE WALKING ENVIRONMENT INFORMATION │───── S121B
└──────────────────────────────────────────────┘
            │
            ▼
┌──────────────────────────────────────────────┐
│    ACQUIRE WALKING TIME INFORMATION    │───── S122
└──────────────────────────────────────────────┘
            │
            ▼
┌──────────────────────────────────────────────┐
│  ESTIMATE HOT/COLD SENSATION BASED ON  │
│    WALKING ENVIRONMENT INFORMATION     │───── S123B
│     AND WALKING TIME INFORMATION       │
└──────────────────────────────────────────────┘
            │
            ▼
┌──────────────────────────────────────────────┐
│  PRESENT COMFORTABLE AREA INFORMATION  │───── S124
└──────────────────────────────────────────────┘
            │
            ▼
┌─────────────────────────┐
│           END           │
└─────────────────────────┘
```

# Fig.47

┌─────────────────────────────────────────────────────────────────────────┐
│                          ⟋ 100                                            │
│                   HOT/COLD SENSATION ESTIMATION DEVICE                     │
│                                                                           │
│      ⟋ 109                              ⟋ 104                              │
│  ┌──────────────────────┐      ┌──────────────────────────┐               │
│  │ PROCESSING CIRCUITRY │      │   COMMUNICATION DEVICE   │                │
│  └──────────┬───────────┘      └────────────┬─────────────┘               │
│             │                               │                             │
│  ───────────┼───────────────┬───────────────┼──────────────┬─────         │
│             │               │               │              │              │
│        ⟋ 105 │               │          ⟋ 106 │              │              │
│      ┌───────┴──────────────┐          ┌──────┴─────────────────┐          │
│      │ INPUT/OUTPUT INTERFACE│          │  POSITIONING DEVICE   │          │
│      └──────────────────────┘          └───────────────────────┘          │
│                                                                           │
└─────────────────────────────────────────────────────────────────────────┘

## INTERNATIONAL SEARCH REPORT

| International application No. |
|---|
| **PCT/JP2023/040365** |

**A. CLASSIFICATION OF SUBJECT MATTER**

*A61B 10/00*(2006.01)i; *A61B 5/11*(2006.01)i; *F24F 11/63*(2018.01)i; *F24F 120/14*(2018.01)n
FI: A61B10/00 X; A61B5/11 200; F24F11/63; F24F120:14

According to International Patent Classification (IPC) or to both national classification and IPC

**B. FIELDS SEARCHED**

Minimum documentation searched (classification system followed by classification symbols)

A61B10/00; A61B5/11; F24F11/63; F24F120/14

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2019-196035 A (DENSO CORPORATION) 14 November 2019 (2019-11-14) abstract, paragraphs [0032]-[0037], [0043]-[0050], [0058], fig. 2-4 | 1-7, 11-18 |
| A | | 8-10 |
| Y | JP 2019-190765 A (MITSUBISHI ELECTRIC CORPORATION) 31 October 2019 (2019-10-31) paragraph [0019] | 1-7, 11-18 |
| Y | WO 2022/059105 A1 (MITSUBISHI ELECTRIC CORPORATION) 24 March 2022 (2022-03-24) paragraphs [0016], [0055]-[0073], [0123]-[0136], fig. 11-15, 29-31 | 1-7, 11-13, 15-18 |
| Y | WO 2021/220391 A1 (MITSUBISHI ELECTRIC CORPORATION) 04 November 2021 (2021-11-04) paragraphs [0082]-[0090], fig. 9 | 1-7, 14-18 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **17 January 2024** | **30 January 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

International application No.

**PCT/JP2023/040365**

### C. DOCUMENTS CONSIDERED TO BE RELEVANT

| Category* | Citation of document, with indication, where appropriate, of the relevant passages | Relevant to claim No. |
|---|---|---|
| A | JP 2020-134068 A (PANASONIC INTELLECTUAL PROPERTY MANAGEMENT CO., LTD.) 31 August 2020 (2020-08-31) <br> entire text, all drawings | 1-18 |
| A | WO 2008/087959 A1 (DAIKIN INDUSTRIES, LTD.) 24 July 2008 (2008-07-24) <br> entire text, all drawings | 1-18 |
| A | WO 2021/192222 A1 (MITSUBISHI ELECTRIC CORPORATION) 30 September 2021 (2021-09-30) <br> entire text, all drawings | 13 |
| A | WO 2019/013014 A1 (MITSUBISHI ELECTRIC CORPORATION) 17 January 2019 (2019-01-17) <br> abstract, paragraphs [0134]-[0167], fig. 21-27 | 14 |

Form PCT/ISA/210 (second sheet) (July 2022)

**INTERNATIONAL SEARCH REPORT**
Information on patent family members

International application No.

**PCT/JP2023/040365**

| Patent document cited in search report | | | Publication date (day/month/year) | Patent family member(s) | | | Publication date (day/month/year) |
|---|---|---|---|---|---|---|---|
| JP | 2019-196035 | A | 14 November 2019 | (Family: none) | | | |
| JP | 2019-190765 | A | 31 October 2019 | (Family: none) | | | |
| WO | 2022/059105 | A1 | 24 March 2022 | (Family: none) | | | |
| WO | 2021/220391 | A1 | 04 November 2021 | US paragraphs [0097]-[0110], fig. 9 | 2023/0108991 | A1 | |
| | | | | EP | 4145055 | A1 | |
| JP | 2020-134068 | A | 31 August 2020 | (Family: none) | | | |
| WO | 2008/087959 | A1 | 24 July 2008 | US entire text, all drawings | 2010/0036533 | A1 | |
| | | | | EP | 2123986 | A1 | |
| | | | | CN | 101583831 | A | |
| | | | | KR | 10-2009-0104063 | A | |
| WO | 2021/192222 | A1 | 30 September 2021 | US entire text, all drawings | 2023/0046536 | A1 | |
| | | | | EP | 4132139 | A1 | |
| WO | 2019/013014 | A1 | 17 January 2019 | US abstract, paragraphs [0233]-[0265], fig. 21-27 | 2020/0134891 | A1 | |
| | | | | EP | 3633280 | A1 | |
| | | | | JP | 2019-20109 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008087959 A